# EUROPEAN PATENT SPECIFICATION

(11) **EP 2 265 954 B1**
(45) Date of publication and mention of the grant of the patent: **29.08.2012**
(21) Application number: 09719558.0
(22) Date of filing: 11.03.2009
(51) Int. Cl.: G01N 33/569, A61K 38/00

(54) **IMMUNOTHERAPEUTIC METHODS AND MOLECULES**
IMMUNTHERAPIEVERFAHREN UND MOLEKÜLE
PROCÉDÉS ET MOLÉCULES D'IMMUNOTHÉRAPIE

(30) Priority: 11.03.2008 GB 0804459
(43) Date of publication of application: 29.12.2010
(73) Proprietor: Cell Medica Limited, London W1T 6ES (GB)
(72) Inventor: TOPP, Max, Stanley, 97070 Würzburg (DE)
(74) Representative: Miles, John Stephen
(86) International application number: PCT/GB2009/000660
(87) International publication number: WO 2009/112831

(56) References cited:
- ROTHBARD JONATHAN B ET AL: "Prediction of peptide affinity to HLA DRB1*0401" INTERNATIONAL ARCHIVES OF ALLERGY AND IMMUNOLOGY, vol. 105, no. 1, 1994, pages 1-7, XP002533375 ISSN: 1018-2438
- BECK OLAF ET AL: "Generation of highly purified and functionally active human T(H)1 cells against Aspergillus fumigatus" BLOOD,, vol. 107, no. 6, 1 March 2006 (2006-03-01), pages 2562-2569, XP002530858 cited in the application
- CATHAL O'BRIAN ET AL.: "Peptide length significantly influences in vitro affinity for MHC class II molecules" IMMUNOME RESEARCH, [Online] vol. 4, no. 6, 26 November 2008 (2008-11-26), pages 1-7, XP002533376 Retrieved from the Internet: URL:http://dx.doi.org/10.1186/1745-7580-4- 6> [retrieved on 2009-06-23]
- RAMADAN ET AL: CLINICAL AND EXPERIMENTAL IMMUNOLOGY, vol. 139, 2005, pages 257-267,
- BANERJEE ET AL: CLINICAL AND EXPERIMENTAL ALLERGY, vol. 31, 2001, pages 761-770,

## Description

The present invention relates to immunotherapeutic methods, and molecules and cells for use in immunotherapeutic methods. In particular, the present invention relates to the immunotherapy of *Aspergillus* infection, including adoptive immunotherapy.

The listing or discussion of an apparently prior-published document in this specification should not necessarily be taken as an acknowledgement that the document is part of the state of the art or is common general knowledge.

Infectious complications are the leading cause of death in immunosuppressed and immunocompromized individuals, including patients receiving allogeneic haematopoietic stem cell transplantation (HSCT). Systemic fungal infections are highly prevalent and also the most detrimental pathogens invading patients^{1,2} with immune deficiencies. *Aspergillus fumigatus* is the most relevant fungus in this subgroup, causing predominantly invasive infections of the oropharynx and the respiratory system with a mortality of up to 80% in some patient cohorts³. With the advance of new treatment modalities for patients otherwise not medically fit to be offered a potentially curative allogeneic HSCT, the incidence of Invasive Aspergillosis (IA) and the mortality attributed thereto has further increased in recent years. This epidemiological change was observed despite the broad use of new antifungal chemotherapy such as voriconazole^{3,4}. Therefore more effective prevention and treatment options are highly warranted for the control of *A. fumigatus* infections in patients undergoing allogeneic HSCT.

*Aspergillus* is a group of around 200 mould species found worldwide. As it is ubiquitously distributed, it is estimated that humans inhale presumably several hundreds of *Aspergillus* conidia every day. Some of the *Aspergillus* species are associated with pathogenic infectious diseases in humans and animals, including *Aspergillus fumigatus, Aspergillus niger, Aspergillus terreus* and *Aspergillus flavus.* Allergic disease is mainly attributed to *A. fumigatus* and *A. clavatus. Aspergillus fumigatus* is a saprophytic, filamentous fungus predominantly found in the soil.

Despite this persistent contact with pathogen, healthy individuals in general do not develop symptomatic *Aspergillus* infections implicating that an effective local immune response must be existent and capable of clearing the infection⁵. The first line of defence in preventing *A. fumigatus* conidia from becoming pathogenic invasive hyphae are alveolar macrophages which phagocytose inhaled conidia and eliminate the pathogen through intracellular reactive oxygen intermediates (ROI)^{6,7}. Should A. *fumigatus* however start to invade the host by initiating germination, the second line of innate defense is activated. Neutrophilic granulocytes adhere to the hyphal wall and release both ROI and hydrolytic enzymes in close proximity to the fungus resulting in irreparable damage to the fungal wall⁶.

In allogeneic SCT recipients multiple adverse factors contribute to the high incidence of *A. fumigatus* infections. Firstly, the respiratory mucosal and epithelial barrier is considerably damaged through the conditioning regimens resulting in more widespread germination of inhaled conidia¹. Secondly, neutrophilic granulocyte counts are transiently reduced in the treated patient resulting in an inferior secondary line of innate defense. Neutrophilic granulocyte counts are reconstituted on average at day 12-15 after transplantation of allogeneic peripheral stem cells, entailing a vastly increased protection against bacterial infection. But despite this early neutrophil reconstitution in allogeneic HSCT recipients, the rate of fungal infections remains high during later months^{3.4}.

One potential explanation for this observation is the prolonged immune suppression conducted for the prevention and treatment of graft-versus-host disease (GvHD), implicating that adaptive immunity may also contribute to the control of IA. This hypothesis is strengthened by numerous murine model systems showing that previous infection with sublethal doses of *A. fumigatus* conidia and other fungal antigens protects mice against lethal rechallenge with the pathogen⁸⁻¹⁰. Furthermore, adoptive transfer of CD4⁺ T cells from immunized animals transfers protective immunity to otherwise susceptible naïve recipient mice, stressing the crucial role of adaptive immunity in protecting the host^{11.12}. In addition to this data, murine experiments applying conditions favoring either a T_{H}1 or T_{H}2 CD4⁺ T cell response revealed that only T_{H}1 CD4⁺ T cells protected the host from lethal challenge with *Aspergillus,* whereas the induction of T_{H}2 responses often exacerbated disease^{8,13}.

The *Aspergillus* f16 allergen has been the focus of studies by others, and immune responses to various peptides have been investigated^{27.28.48}.

Recent work by the inventors' group utilizing crude *A. fumigatus* extract as well as work done by other groups, demonstrated that healthy humans also exhibit a memory response for this pathogenic fungus¹⁴⁻¹⁷. As in rodents, the T cell response is dominated by CD4⁺ T cells with a T_{H}1-biased phenotype. However, it has so far not been possible to turn these basic findings into clinical applications because the memory T cell population specific for *A. fumigatus* has not been further characterized by identification of immunogenic antigens.

Human diseases associated with *Aspergillus* include Allergic Bronchopulmonary Aspergillosis (ABPA), Invasive Aspergillosis (IA), Aspergilloma, and Chronic *Aspergillus* sinusitis. ABPA is caused by the presence of *Aspergillus* in the bronchopulmonary region leading to inflammation and allergic responses. Bronchiectasis and long-term lung degeneration develops with symptoms similar to asthma. Invasive Aspergillosis is associated with immune deficiencies. The infection usually begins in the lungs and may transfer to other organs. The mortality rate associated with IA is high. Aspergilloma results when other conditions create cavities in the lung which allow the *Aspergillus* spores to germinate and to colonize the area, forming a fungal ball. The disease is often chronic and incurable. Chronic *Aspergillus* sinusitis develops in the sinus in a similar manner to aspergilloma in the lungs. There is increasing belief that many chronic sinus problems are a result of allergic reactions to fungus.

Ramadan et al (2005) Clin. Exp. Immunology 139, 257-267 describes the generation of Th1 T cell responses directed to a HLA Class II restricted epitope from the *Aspergillus* f16 allergen.

Banerjee et al (2001) Clin. Exp. Allergy 31, 761-770 describes the cloning and expression of *Aspergillus fumigatus* allergen Asp f16.

We present data on the identification of the HLA context in which an immunodominant epitope of the Asp f16 antigen is presented for immune system recognition in humans. This is HLA-DRB1*04, the most abundant MHC class II allele in the human population and we demonstrate that such epitope-specific T cells can be activated by antigen-presenting cells fed with germinating *A. fumigatus* conidia or hyphae. Furthermore we also demonstrate that innate immune cells can be conditioned by *Aspergillus-specific* T cell immunity resulting in enhanced killing of *Aspergillus fumigatus* by innate effector cells.

Surprisingly, we have found that CD4⁺ T cells specific for the HLA-DRB1*04 restricted peptide containing the epitope or an immunogenic fragment thereof are specifically activated by germinating *A. fumigatus* conidia and hyphae, whereas resting conidia have only limited stimulatory capacity and other fungal pathogens like *Candida albicans* fail to induce activation.

The *Aspergillus*-specific CD4⁺ T cells are useful in adoptive T cell therapy which may be of benefit in patients with evidence of *Aspergillus*-related diseases, or for prophylactic or preemptive therapy, for example in asymptomatic HSCT recipients. The discovery of the HLA restriction of the FHT immunodominant peptide allows the identification of the specific patients that can benefit from the use of this peptide in immunotherapy protocols. In addition to this any cell product manufactured using this peptide can utilize the discovery of the HLA restriction to enumerate and define the cell product as well as track the Aspergillus specific **T** cells directly *ex vivo* by using techniques such as MHC multimer technology as discussed below.

The following peptides, polypeptides, polynucleotides, expression vectors and host cells are useful in the practice of the invention.

A first aspect of the invention provide a peptide of less than 5000 molecular weight comprising the amino acid sequence FHTYTIDWTKDAVTW, wherein the peptide is capable of binding HLA-DRB1 04 and wherein when bound to HLA-DRB1 04 the peptide-bound HLA-DRB1 04 is capable of identifying T cells specific for *Aspergillus fumigatus.*

The specific peptide with the amino acid sequence FHTYTIDWTKDAVTW has been called the FHT peptide, and has been shown to bind to HLA-DRB1 *04.

HLA-DRB1*04 includes the sub-types DRB1*0401, DRB1*0402, DRB1*0403, DRB1*0404 and DRB1*0408. For the avoidance of doubt we include all of these in the term DRB 1*04.

The peptide of the invention will bind HLA-DRB1 *04 which is one of the most prevalent Class II HLA alleles in the human population.

Whether or not a peptide binds to HLA-DRB1*04 may be determined using any method known in the art. A suitable method is shown in Example 1, with particular reference to Figure 2B. One method is to introduce the peptide into PBMCs taken from an HLA-DRB1*04 human and determine whether an activating or proliferative response is obtained which is indicative of binding. Binding to HLA-DRB1*04 may also be assessed using the methods described in Figures 7 and 8.

The recognition of harmful pathogens or disease causing mutations within self tissue occurs through two mechanisms within the human immune system. Antibody molecules expressed by B cells bind with biological molecules, typically expressed on the surface of invading microorganisms or deviant self cells, in highly specific manner and will label these molecules in a manner which will trigger an appropriate immune response. In addition to the antibody response, pathogens and disease causing mutations can be detected due to unique proteins expressed by the pathogens or mutations. These proteins are broken down into small peptide fragments by natural and continuous protein degradation systems in the human cell. The peptide fragments will bind with special molecules, referred to as MHC Class I and MHC Class II molecules, expressed on the surface of all cells. In the human, these molecules are referred to as HLA molecules and are numbered according the large number of alleles which exist across the human population.

In the human, the peptide fragments derived from pathogens bind with specific HLA molecules and are transported to the surface of the cell. The peptides are captured in a particular configuration which allows the peptides to be detected by T cell receptors ("TCRs") expressed on the surface of T cells. Through natural selection and development processes which are linked to the immune system's ability to detect danger signals in connection with the presence of a foreign organism, the human body produces T cells with TCRs which can recognize and distinguish between peptide fragments derived from a harmful pathogen and peptides which are derived from harmless microorganisms or healthy self tissue.

MHC Class I molecules present peptides derived mainly from proteins found within the cell to CD8+ T cells, also referred to as cytotoxic T cells or CTL. The peptides which bind with MHC Class I molecules are usually 8-10 amino acids in length. MHC Class II molecules present peptides derived from proteins or organisms which have been endocytosed from the extracellular milieu. MHC Class II molecules present peptides to CD4+ T cells, also referred to as T helper cells although CD4+ T cells may also have direct cytotoxic functions. The peptides which bind to MHC Class II molecules are relatively unconstrained in terms of length, although Class II peptides generally fall within a range of 13 -17 amino acids, for example 14 or 15 or 16 amino acids.

A particular advantage of a peptide of the invention, particularly the FHT peptide, is that it binds to the HLA-DRB1*04 molecule which is presented on a very prevalent HLA allele across the patient population, hence it is possible to treat many more patients than a peptide which fits a rare HLA allele. Amongst individuals expressing HLA DRB1*04, responding T cells were found in all cases tested which indicates that the FHT peptide represents an immuno-dominant epitope.

By "peptide" we include not only molecules in which amino acid residues are joined by peptide (-CO-NH-) linkages but also molecules in which the peptide bond is reversed. Such retro-inverso peptidomimetics may be made using methods known in the art, for example such as those described in Mézière et al (1997) J. Immunol. 159, 3230-3237. This approach involves making pseudopeptides containing changes involving the backbone, and not the orientation of side chains. Mézière *et al* (1997) show that, at least for MHC class II and T helper cell responses, these pseudopeptides are useful. Retro-inverse peptides, which contain NH-CO bonds instead of CO-NH peptide bonds, are much more resistant to proteolysis.

Similarly, the peptide bond may be dispensed with altogether provided that an appropriate linker moiety which retains the spacing between the Cα atoms of the amino acid residues is used; it is particularly preferred if the linker moiety has substantially the same charge distribution and substantially the same planarity of a peptide bond.

It will be appreciated that the peptide may conveniently be blocked at its N- or C-terminus so as to help reduce susceptibility to exoproteolytic digestion. Similarly, it will be appreciated that the peptide of the invention may be in salt form or may contain additional esters of -OH or -COOH groups or amides of -NH₂ groups. The peptides of the invention are defined in the claims. The following definitions of "portion of at least II amino acids", and "variant" are used with respect to the HLA-peptide complex of the invention.

By a "portion of at least II amino acids" of the given amino acid sequence we mean at least 11 or 12 or 13 or 14 consecutive amino acids of the given sequence such that the peptide containing the portion and preferably the portion itself, is still able to bind to the HLA molecule in substantially the same way as a peptide consisting of the given amino acid sequence or the FHT peptide. Preferably, the peptide comprising the portion is able to bind to the HLA molecule in substantially the same way as a peptide consisting of the given amino acid sequence.

By a "variant" of the given amino acid sequence we mean that the side chains of one or two or three of the amino acid residues are altered (for example by replacing them with the side chain of another naturally occurring amino acid residue or some other side chain) such that the peptide is still able to bind to the HLA molecule in substantially the same way as a peptide consisting of the given amino acid sequence. For example, a peptide may be modified so that it at least maintains, if not improves, the ability to interact with and bind HLA-DRB1*04. and so that it at least maintains, if not improves, the ability to generate activated CD4* T cells which can recognise *Aspergillus fumigatus.* Typically, the amino acid alternatives are conservative in nature, such as from within the groups Gly, Ala; Ile, Leu, Val; Ser, Thr; Tyr, Phe, Trp; Glu, Asp; Gln, Asn, His, Met, Cys, Ser.

Peptides of at least 15 amino acids are preferred. Thus, the invention also includes peptides of 16 or 17 or 18 or 19 or 20 or 21 or 22 or 23 or 24 or 25 amino acids that contain the amino acid sequence FHTYTIDWTKDAVTW. As noted above, the peptides of the invention are capable of binding HLA-DRB1 *04.

Those amino acid residues that are not essential to interact with the T cell receptor can be modified by replacement with another amino acid whose incorporation does not substantially affect T cell reactivity and does not eliminate binding to the relevant HLA allele.

The peptides of the invention (and for use in the invention) are less than 5 000 and typically about 4 000 or 3 000 or 2 000. In terms of the number of amino acid residues, the peptides of the invention may have fewer than 30 or 20 or 19 or 18 or 17 or 16 or 15 or 14 or 13 or 12.

It will be appreciated from the following that in some applications the peptides of the invention may be used directly (ie they are not produced by expression of a polynucleotide in a patient's cell or in a cell given to a patient); in such applications it is preferred that the peptide has fewer than 30 or 25 or 24 or 23 or 22 or 21 or 20 or 19 or 18 or 17 or 16 or 15 or 14 or 13 or 12 residues.

The peptides of the invention are able to bind to HLA-DRB1*04. It is particularly preferred if the peptides bind selectively to HLA-DRB1*04.

It is further preferred that the peptides of the invention are ones which can be used to generate peptide-specific CD4⁺ T cells which mediate specific killing of *Aspergillus fumigatus* and in particular germinating *A. fumigatus* conidia and hyphae either through direct cytotoxic effector functions or through enhancement of innate immune functions (see Examples).

The peptides of the invention are particularly useful in immunotherapeutic methods to combat *Aspergillus* infection. In particular, the peptide in combination with the specific HLA molecule can be used to select and define appropriate T cells, and trace them once they are put into the patient, as discussed below. It is particularly preferred that in all the immunotherapeutic methods of the invention that the patient to be treated is one who carries Class II HLA-DRB1*04 (ie has a Class II HL4-DRB1*04-positive genotype), and has antigen presenting cells which express HLA-DRB1*04.

The peptides of (and for use in) the invention are ones which bind HLA-DRB1*04 and when so bound the HLA-DRB1*04-peptide complex, when present on the surface of a suitable antigen-presenting cell, is capable of eliciting a T cell mediated immune response which mediates or helps to mediate the immune systems attack on *Aspergillus fumigatus.* In particular, the production of cytokines by a CD4⁺ T cell may mediate the attack on *A. fumigatus.*

It is well known that an optimum length for a peptide to bind to an HLA Class II molecule is around 13 to 17 amino acids, such as 13, 14, 15, 16 or 17, preferably 15 amino acids.

A particular preferred peptide for use in the methods, pharmaceutical formulations and medicaments of the invention consists of the amino acid sequence FHTYTIDWTKDAVTW.

If a peptide which is greater than around 15 amino acid residues is used directly to bind to a Class II HLA molecule, it is preferred that the residues that flank the core HLA binding region are ones that do not substantially affect the ability of the peptide to bind to the HLA molecule or to present the peptide to an Aspergillus-specific T cell. However, it will be appreciated that larger peptides may be used, especially when encoded by a polynucleotide, since these larger peptides may be fragmented by suitable antigen-presenting cells.

Peptides (at least those containing peptide linkages between amino acid residues) may be synthesised using any method well known in the art, for example by the Fmoc-polyamide mode of solid-phase peptide synthesis as disclosed by Lu et al (1981) J. Org. Chem. 46, 3433 and references therein. Reagents for peptide synthesis are generally available from Calbiochem-Novabiochem (UK) Ltd, Nottingham NG7 2QJ, UK as well as many other commercial providers of chemical and biological reagents. Purification may be effected by any one, or a combination of, techniques such as size exclusion chromatography, ion-exchange chromatography and (principally) reverse-phase high performance liquid chromatography. Analysis of peptides may be carried out using thin layer chromatography, reverse-phase high performance liquid chromatography, aminoacid analysis after acid hydrolysis and by fast atom bombardment (FAB) mass spectrometric analysis.

As is discussed in more detail below, these peptides may be used to create MHC multimers whereby the peptide is able to bind to and therefore stabilises the specific HLA-DRB1*04 molecule. This enables the HLA molecule to be multimerised and conjugated to a fluorescent molecule or a magnetic bead. This multimer can then recognise and bind directly to an *Aspergillus* specific T cell via the TCR. By using this technique cells can be directly selected from fresh or cultured cells or enumerated in both cell therapy products and in blood samples from patients having received a cell therapy product. It is impossible to use this MHC multimer technology only with knowledge of the peptide, as the T cell will only bind to the peptide which is displayed in the context of the correct HLA molecule.

It will be appreciated that the peptide is typically used in an HLA-DRB1*04-specific context.

A second aspect of the invention provides a polypeptide which is a fusion of a peptide of the invention and another peptide as specified in the claims. The other peptide may be fused to the N-terminus of the peptide of the invention or to the C-terminus of the peptide of the invention or, in some embodiments other peptides (either the same or different) may be fused to both the N-terminus or C-terminus of the peptide of the invention. Typically, these peptides may be useful in ensuring the FHT Peptide is directed through the endosomal pathway for MHC Class II presentation. Alternatively, these fused peptides may be linked to known immunogenic substances such that the combined peptide structure is more effective in inducing an effective human immune response against *Aspergillus.*

The peptide of the invention may be comprised within, or fused to, an HLA molecule such that the peptide can occupy the peptide-binding groove of the HLA molecule. Fusion molecules of this type, although using a different peptide, have been synthesised by Mottez et al (1995) J. Exp. Med. 181, 493-502. Preferably, the HLA molecule to which the peptide of the invention is fused is HLA-DRB1*04.

A preferred fusion polypeptide of the invention is between a peptide of the invention and the MHC Class II invariant chain which stabilises the MHC Class II molecule and which is involved in directing the HLA molecule through the endosomal pathway leading to expression on the surface of a cell.

It will be appreciated that the polypeptide is typically used in an HLA-DRB1*04-specific context.

A third aspect of the invention provides a polynucleotide encoding a peptide as defined in the first aspect of the invention or a polypeptide as defined in the second aspect of the invention. The polynucleotide may be DNA or RNA and it may or may not contain introns so long as it codes for the peptide. Of course, it is only peptides which contain naturally occurring amino acid residues joined by naturally-occurring peptide bonds which are encodable by a polynucleotide.

It will be appreciated that the polynucleotide is typically used in an HLA-DRB1*04-specific context.

A fourth aspect of the invention provides an expression vector capable of expressing a peptide according to the first or aspect of the invention or a polypeptide according to the second aspect of the invention.

It will be appreciated that the expression vector is typically used in an HLA-DRB1*04-specific context.

Methods for manipulating, changing and cloning nucleic acid molecules are well known in the art, for example Sambrook J and Russell, DW, Molecular Cloning, A Laboratory Manual, 3rd Edition, 2001, Cold Spring Harbor Laboratory Press describes such techniques including PCR methods.

Suitable expression vectors include viral based vectors such as retroviral or adenoviral or vaccinia virus vectors or lentiviral vectors or replication deficient MV vectors.

Suitable general cloning vectors include plasmids, bacteriophages (including λ and filamentous bacteriophage), phagemids and cosmids.

Suitable host cells include bacteria, yeast, insect and mammalian cells. Particular bacteria include *Escherichia coli, Bacillus subtilis* and *Salmonella typhimurium.*

Particular yeast cells include *Saccharomyces cerevisiae* and *Schizosaccharomyces pombe.*

Particular mammalian cells include CHO cells, COS cells and other mammalian cells such as antigen presenting cells.

It will be appreciated that certain host cells of the invention are useful in the preparation of the peptides or polypeptides of the invention, for example bacterial, yeast, mammalian and insect cells. Thus, a fifth aspect of the invention provides a host cell comprising a polynucleotide of the invention or an expression vector of the invention. It is particularly preferred that the host cell is a human host cell, such as an antigen presenting cell, which carries Class II HLA-DRB1*04.

A sixth aspect of the invention provides a method of producing a peptide of the first aspect of the invention or a polypeptide of the second aspect of the invention, the method comprising culturing host cells which contain a polynucleotide or expression vector which encodes the peptide or polypeptide and obtaining the peptide or polypeptide from the host cell or culture medium. The peptides and polypeptides of the invention may also be chemically synthesised as discussed above.

The peptides of the invention may be used in the production of T cells, particularly CD4⁺ T cells, specific for the *Aspergillus fumigatus* f16 polypeptide and in particular for a peptide which has the amino acid sequence FHTYTIDWTKDAVTW or a portion thereof, for example a portion with from 11 or 12 or 13 or 14 contiguous amino acids, and which may be presented by HLA-DRB1*04.

Thus, further aspects of the invention provide compositions which comprise a peptide according to the first aspect of the invention or a polypeptide according to the second aspect of the invention which compositions are suitable for raising T cell clones, particularly CD4⁺ T cell clones, specific for the peptides, in particular the FHT peptide, when presented by HLA-DRB1*04. Such compositions are typically sterile and pyrogen free. The composition is typically a pharmaceutical composition which contains a pharmaceutically acceptable carrier. Typically, for the generation of T cells, particularly CD4⁺ T cells, the peptides are used in the range 200 µM to 1 nM.

Typically, the composition is an aqueous composition. In some instances it may be desirable to include a peptide-solubilising agent such as DMSO in the composition.

It will be appreciated that the polynucleotide or expression vectors of the invention may also be provided in a pharmaceutical composition containing a pharmaceutically acceptable carrier. Typically, these compositions would be sterile and pyrogen free.

The peptides, polypeptides, polynucleotides and expression vectors may be packaged and presented for use as a medicament. In particular, they are of use in combating *Aspergillus* infection. By "combating *Aspergillus* infection" we include treating patients who have an *Aspergillus* infection, for example patients who have ABPA or Invasive Aspergillosis (IA) or Aspergilloma or Chronic *Aspergillus* sinusitis. We also include administering the peptides, polypeptides, polynucleotides and expression vectors (either alone or in combination with, or present in or on, a suitable HLA matched antigen presenting cell such as a dendritic cell or B cell or monocytes or a synthetic APC) to not only patients who have an *Aspergillus* infection, but also to those at risk of *Aspergillus* infection. Patients at risk of *Aspergillus* infection include those who are immunocompromised or immunodepleted such as those undergoing allogeneic HSCT, organ transplant patients, autoimmune patients receiving immunosuppressive drugs, patients with genetic immune disorders, AIDS patients, or patients undergoing chemotherapy for cancer or leukaemia patients. Thus, it will be appreciated that "combating" includes preventing (or helping to prevent) *Aspergillus* infection and treating a patient prophylactically.

Allergy to *Aspergillus,* in particular to *A. fumigatus,* is a medical problem. The invention also includes the use of the peptides, polypeptides, polynucleotides and expressions for combating *Aspergillus* allergy, particularly allergy to *A. fumigatus.* By "combating allergy to *Aspergillus"* we include treating allergy or preventing allergy. The peptides of the invention are particularly useful in this regard.

The *Aspergillus* infection may be an infection with any *Aspergillus* spp, and in particular an *Aspergillus* spp or strain which includes the f16 protein in the fungal proteome, and more particularly includes the peptides of the invention, particularly the FHT peptide as a part thereof. Typically, the patient has or is at risk of obtaining an infection of *Aspergillus fumigatus.* Preferably, the patient is HLA-DRB1*04-positive.

The invention also includes a method of combating Aspergillus infection in a patient, the method comprising administering to the patient an effective amount of a peptide or polypeptide or polynucleotide or expression vector of the invention (either alone or in combination with, or present in or on, a suitable antigen presenting cell such as a dendritic cell or B cell or T cell or monocyte) wherein the amount of the peptide or polypeptide or polynucleotide or expression vector is effective to provoke an anti-*Aspergillus* response in said patient. Preferably, the patient is HLA-DRB1*04-positive. Preferably, the antigen-presenting cell is HLA-DRB1*04-positive. The efficacy of this method of using the invention can typically be measured by testing for the presence of *Aspergillus*-specific T cells in a patient who otherwise is not able to generate an immune response.

The anti-*Aspergillus* response may be measured by Elispot, intracellular cytokine staining or by a proliferation assay, or by using MHC multimer technology.

A still further aspect of the invention includes a kit of parts comprising a peptide according to the invention or a polypeptide according to the invention or a polynucleotide according to the invention or an expression vector according to the invention and an antigen presenting cell. The kit of parts is useful in the preparation of activated *Aspergillus*-specific T cells as described below. Preferably, the antigen-presenting cell is HLA-DRB1*04 positive.

A yet still further aspect of the invention includes an antigen-presenting cell wherein its HLA Class II molecules are loaded with a peptide of the invention. Typically, in all relevant embodiments of the invention the antigen-presenting cell contains an HLA-DRB1*04 molecule which presents the peptide.

Preferred antigen-presenting cells are dendritic cells or B cells or monocytes derived from the donor in the case of the treatment of patients undergoing allogeneic HSCT, and for other treatments the antigen-presenting cells are preferably autologous dendritic cells or B cells or T cells (ie derived from the patient to be treated).

The antigen presenting cells may be loaded with the peptide *in vitro* as described in detail below. Alternatively, the antigen presenting cell may be a recombinant cell which expresses the peptide or polypeptide of the invention from a suitable polynucleotide or expression vector.

The antigen presenting cell, such as a dendritic cell, a B cell or a monocyte which is presenting the peptide or polypeptide may be used as a vaccine and may be packaged and presented for use as a medicament. The antigen presenting cell which is presenting the peptide may be prepared as a pharmaceutical composition (ie by preparing it in a pharmaceutically acceptable carrier) and it may also be packaged and presented for use in combating *Aspergillus* infection in a patient. Preferably, the antigen presenting cell isautologous, or is compatible with the patient to whom it is to be administered based upon a close HLA match.

The invention therefore also includes a method for combating *Aspergillus* infection in a patient, the method comprising administering to the patient an effective amount of an antigen presenting cell which is loaded with the peptide wherein the amount of said antigen presenting cell is effective to provoke an anti*-Aspergillus* response in said patient. Preferably, the patient is one who carries Class II HLA-DRB1*04. Preferably the antigen presenting cell is one which carries Class II HLA-DRB1*04, and presents the peptide. The antigen presenting cells may be used in cell doses from 10² to 10⁷ cells per kg of the patient's weight, depending on whether the cells are autologous or only HLA matched. In this aspect of the invention, antigen presenting cells may be infused to switch on T cells *in vivo.*

T cells are switched on by antigen-presenting cells such as dendritic cells. Hence cell therapy can involve T cells that have been switched on *ex vivo* and then infused into the patient.

Thus, a further aspect of the invention provides a method for selecting *Aspergillus-*specific T cells, the method comprising contacting a population of T cells with a peptide or polypeptide of the invention presented in a Class II HLA-DRB1*04 molecule to which said peptide binds.

Preferably, the population of T cells are from an individual who has been exposed to *Aspergillus,* in particular *A. fumigatus.*

The peptide of the invention may be used to generate an expansion of T cells specific for *Aspergillus* for patients who are HLA-DRB1*04 positive and there are several ways in which the invention may be used.

As mentioned previously the knowledge that the peptide binds the HLA-DRB1*04 molecule and is only recognised by responder T cells in this context means that the creation of MHC multimers that will directly bind the T cells can occur. This reagent can be used to directly select the *Aspergillus* specific cells from the bulk culture in order to infuse cells with a high purity into a patient.

The invention also includes use of the MHC multimer in conjunction with additional multimers of other specificities (to be defined) to fully characterise the product prior to infusion to ensure purity and safety of the product. Following infusion of *Aspergillus* T cells to the patient the reconstitution of Aspergillus specific immunity could be monitored in the patient using the FHT/ HLA-DRB1*04 multimer directly *ex vivo.*

An additional application of this technology would be that instead of relying on the innate antigen presentation ability of the patient's own cells to present the peptide of the invention in order to expand antigen presenting cells, an artificial antigen presenting cell could be used which consists of either cell lines deficient in all MHC but HLA-DRB1*04 pulsed with the peptide or artificial antigen presenting cells which can are engineered to supply the co-stimulation required for T cell expansion along with the HLA-DRB1*04 peptide combination in order to stimulate and expand the *Aspergillus* specific T cells. These embodiments of the invention are discussed in more detail below.

Suitable methods for selecting *Aspergillus*-specific T cells include the use of ELISPOT analysis to confirm the responding T cells, as described in Example 1. Blood is obtained from HLA-DRB1*-typed donors or patients. Peripheral blood mononuclear cells (PBMC) are isolated via centrifugation in Biocoll Separating Solution (Biochrom, Berlin, Germany) and either used directly after preparation or cryopreserved for later use. Cells are cultured in RPMI 1640 with L-Glutamine (Invitrogen, Karlsruhe, Germany), supplemented with 10% heat-inactivated, pooled human serum and 100 U/ml Penicillin-Streptomycin (invitrogen, Karlsruhe, Germany). *Aspergillus*-specific T cell lines are generated by incubation of 1x10⁷ whole PBMC. per well in 6-well culture plates with the FHT peptide antigen for 7 days. Lymphocyte cultures are supplemented with 5 U/ml IL-2 (Proleukin, Chiron, Ratingen, Germany) every other day and culture medium replenished as needed. The T cell clones are generated by stimulating PBMCs repeatedly with 1 ua/ml FHT peptide once weekly for 4 weeks. Subsequently T cell clones are generated by limiting dilution in 96-well plates and expanded using the rapid expansion protocol as described by Beck *et al*¹⁴. This small scale culture system may be scaled up and adapted to a "closed system" whereby clinical grade T cells suitable for infusion back into patients can be generated.

Suitable methods for selecting *Aspergillus*-specific T cells include the use of a fluorescence-activated cell sorter (FACS). Following exposure of the donor or patient PBMCs to the FHT peptide, the responding T cells are labeled based on activation markers such as the upregulation of CD69 or by behavioural characteristics such as the secretion of IFN-γ. The labeling is achieved using an antibody specific for the activation marker or the secreted cytokine and such antibody is conjugated to a fluorochrome. The cells can then be separated and selected through a flow cytometer equipped for FACS analysis.

Alternatively, labeling of the responding T cells is based on the binding of an MHC multimer (HLA-DRB1*04), which is conjugated to a fluorescent marker, to the specific TCR on the surface of the *Aspergillus*-specific T cell.

Suitable methods for selecting T cells include the Cytokine Secretion Assay System which is manufactured by Miltenyi Biotec and involves four key steps: 1) exposure of PBMCs (which contain antigen-presenting cells) from a blood sample to an immunogenic antigen (any antigen, but in the present invention would be a peptide of the invention, in particular the FHT peptide); 2) the responding *Aspergillus* (eg FHT peptide)-specific T cells begin to secrete IFN-γ which is associated with an active and effective T cell immune response and these responding T cells are labelled with a bi-specific catch antibody which simultaneously binds to CD45 (a T cell marker) and IFN-γ; 3) a second antibody then labels the captured IFN-γ and in so doing labels the T cell which has secreted the IFN-γ this second antibody is also conjugated to a magnetic bead; 4) the cells are passed through a magnetic column and the responding T cells (which recognised the FHT Peptide as evidenced by the secretion of IFN-γ are retained in the column by the attached magnetic bead. The non-labelled cells are washed through and then the magnetic field is switched off and the labelled cells are released and collected as the positive fraction.

Methods for making and using peptide-loaded MHC multimers are described, for example, in Altman et al (1996) Science 274, 94-96; Kuabel et al (2002) Nature Medicine 8, 631-637; and Neudorfer et al (2007) J. Immunol. Methods 320, 119-131.

The purity of a T cell population may be assessed using the fluorescently labelled MHC multimer/peptide complex as discussed above.

Suitable methods for selecting T cells also include the MHC Multimer System, available through Proimmune and Stage Pharmaceutical, which works by creating an artificial construction of Class II HLA molecules which bind, in the present case, the peptide of the invention (eg the FHT peptide). These soluble, standalone HLA molecules may be constructed in a multimeric configuration so that a single multimer has 4-5 HLA molecules each loaded with a peptide of the invention such as FHT peptide. These multimers may also be attached to a magnetic bead as above. The multimers are released into a blood sample, and the HLA:peptide construct will bind with T cell receptors that recognise the FHT Peptide and hence will label the T cells which will recognise and mount an immune response against *Aspergillus.* The cell sample is passed through a magnetic column, and the labelled cells are retained and then released as described above.

It will be appreciated from the above that the invention includes a complex comprising a Class II HLA-DRB1*04 molecule bound to a peptide according to the first aspect of the invention. Conveniently, the complex is a soluble complex and is not cell-bound. Preferably, the Class II molecule is a MHC multimer, such as those discussed above. Preferably, the peptide in the complex is the FHP peptide, but may be any other peptide of the invention that will form a complex, and be useful in eliciting an anti-*Aspergillus* T cell response. As is plain, the complex may be used for isolating *Aspergillus-specific* T cells as discussed above. The complex may also be used to identify an *Aspergillus-*specific T cell in a sample.

Thus, the Class II HLA-DRB1*04 molecule which presents the peptide may be present on the surface of an antigen-presenting cell, for example which is present in PBMC, or it may be a synthetic soluble Class II HLA molecule.

Conveniently, the antigen is loaded into class II HLA molecules expressed on the surface of a suitable antigen-presenting cell by contacting a sufficient amount of the peptide of the invention, for example the FHT peptide, with an antigen-presenting cell in the absence of other peptides which may complete in binding the target HLA allele. Conveniently, the antigen presenting cell may be transfected with a polynucleotide or expression vector of the invention, and the cell machinery loads suitable peptides into the class II HLA molecules for presentation.

Preferably, the *Aspergillus*-specific T cells are CD4⁺, such as CD4⁺ T_{H}1 cells, or are T regulatory cells (which are particularly relevant for combating allergy) T_{H}1 helper cells may be converted to T regulatory cells by exposure to a CD28 antibody (superagonist) CD8⁺ cells. Typically, then, the cells are CD3⁺ CD4⁺ and/or CD25⁺ and/or Foxp3⁺ and/or GITR⁺ and/or CD127⁺.

In one embodiment the antigen is linked to the MHC Class II molecule expressed on the surface of the antigen presenting cell with a suitable flexible linker such that the peptide can occupy the MHC Class II binding groove, or alternatively the antigen is linked to the Invariant Chain which stabilises the MHC Class II molecule and is involved in directing the molecule through the endosomal pathway common to Class II presentation of peptides. Thus, the antigen presenting cells may be cells which contain a polynucleotide (eg expression vector) encoding the fusion polypeptide discussed above.

The antigen presenting cell may be genetically engineered to ensure the expression of the HLA DRB1*04 molecule on its own or in combination with the peptides or polypeptides of the invention or in combination with the peptides of the invention or polypeptides of the invention and co-stimulatory molecules which are useful in enhancing the immunogenicity of the peptide and the resulting ability to produce *Aspergillus*-specific T cells. The antigen-presenting cells may be exposed to various cytokines such as IL-2, IFN-γ, and TNF-α, which are known to activate T cells and to direct a T_{H}1 response.

The *Aspergillus*-specific T cell is isolated for further use. With some techniques it is possible to isolate a sufficient number of specific T cells for therapeutic use directly, but it may be necessary to expand or clone the T cells to produce a sufficient number. For adoptive immunotherapy it is generally preferred to use a technique which allows for the isolation of sufficient numbers of cells directly since this can be achieved within a day (whereas cell expansion may take several weeks).

A suitable procedure for identifying pathogen-specific donor clones is described in Perruccio et al (2005) Blood 106, 4397-4406.

The *Aspergillus*-specific T cells which are directed against the peptides of the invention are useful in therapy. Thus, a further aspect of the invention provides *Aspergillus-*specific T cells obtainable by the foregoing methods of the invention.

A still further aspect of the invention provides an *Aspergillus*-specific T cell which is able to recognize the HLA-DRB1*04-presented FHT peptide. Typically, this T cell is CD3⁺. It may be CD3⁺CD4⁺ or it may be CD3⁺CD8⁺. Typically, this T cell will produce cytokines and proliferate when in the presence of the FHT peptide when presented by HLA-DRB1*04, for example when presented by an antigen presenting cell which expresses HLA-DRB1*04.

The activated *Aspergillus*-specific T cells of the invention may be packaged and presented for use as a medicament. The invention also includes a pharmaceutical preparation comprising *Aspergillus*-specific T cells of the invention and a pharmaceutically acceptable carrier. Typically, the carrier is sterile and pyrogen free. The invention also includes the Aspergillus-specific T cells packaged and presented for use in combating *Aspergillus* infection. Preferably, the patients to be treated are ones which carry Class II HLA-DRB1*04.

For the treatment of patients following allogeneic HSCT, the *Aspergillus*-specific T cells typically are isolated from the donor providing the stem cells. If the number of *Aspergillus*-specific T cells is not sufficient through direct recovery of the cells from the donor, then the cells may be expanded through well established cell proliferation techniques.

However, in some embodiments the T cells are autologous (ie from the patient), and in some embodiments the T cells may be from an unrelated donor, for example from a bank of T cells which have been HLA-typed.

The activated *Aspergillus*-specific T cells may be stored in any suitable way, for example by cryopreservation in medium containing serum and DMSO as is well known in the art.

The methods of the invention therefore include methods of adoptive immunotherapy.

it will be appreciated that the MHC multimers loaded with peptide (HLA-DRB1*04-peptide complex of the invention) may be used to assess whether a blood sample contains *Aspergillus*-specific T cells. This may be done in a sample from a patient before treatment and/or during the course of treatment or after treatment.

The *Aspergillus*-specific T cells contain a T cell receptor (TCR) which is involved in recognising cells which express the polypeptide. It is useful if the cDNA encoding the TCR is cloned from the *Aspergillus*-specific T cells and transferred into autologous or donor-derived T cells for expression, thereby obviating the need to find naturally occurring *Aspergillus*-specific T cells expressing the TCR.

The TCRs of *Aspergillus*-specific T cell clones of the invention specific for the peptides of the first aspect of the invention as presented by HLA-DRB1*04 are cloned. The TCR usage in the *Aspergillus*-specific T cell clones is determined using (i) TCR variable region-specific monoclonal antibodies and (ii) RT-PCR with primers specific for Vα and Vβ gene families. A cDNA library is prepared from poly-A mRNA extracted from the *Aspergillus*-specific T cell clones. Primers specific for the C-terminal portion of the TCR α and β chains and for the N-terminal portion of the identified Vα and β segments are used. The complete cDNA for the TCR α and β chain is amplified with a high fidelity DNA polymerase and the amplified products cloned into a suitable cloning vector. The cloned α and β chain genes may be assembled into a single chain TCR by the method as described by Chung et al (1994) Proc. Natl. Acad Sci. USA 91, 12654-12658. In this single chain construct the VαJ segment is followed by the VβDJ segment, followed by the Cβ segment followed by the transmembrane and cytoplasmic segment of the CD3 ξ chain. This single chain TCR is then inserted into a retroviral expression vector (a panel of vectors may be used based on their ability to infect mature human CD4⁺ T lymphocytes and to mediate gene expression: the retroviral vector system Kat is one preferred possibility (see Finer et al (1994) Blood 83, 43). High titre amphotrophic retrovirus are used to infect purified CD4⁺ T lymphocytes isolated from the peripheral blood of tumour patients following a protocol published by Roberts et al (1994) Blood 84, 2878-2889. Anti-CD3 antibodies are used to trigger proliferation of purified CD4⁺ T cells, which facilitates retroviral integration and stable expression of single chain TCRs. The efficiency of retroviral transduction is determined by staining of infected CD4⁺ T cells with antibodies specific for the single chain TCR.

Patients may be treated with in between 10⁶ to 10⁸ (most likely 10⁷) autologous, transduced *Aspergillus-specific* T cells.

Other suitable systems for introducing genes into **T** cells are described in Moritz et al (1994) Proc. Natl. Acad. Sci. USA 91, 4318-4322. Eshhar et al (1993) Proc. Natl. Acad. Sci. USA 90, 720-724 and Hwu et al (1993) J. Exp. Med. 178, 361-366 also describe the transfection of T cells.

Thus, a further aspect of the invention provides a TCR which recognizes a peptide of the invention, in particular the FHT peptide, on a HLA-DRB1*04 molecule. Typically, the peptide is presented on a dendritic cell or monocyte which present the antigen to T cells. The T cells then secrete cytokines which in turn activate monocytes and neutrophils for enhanced killing of *Aspergillus.*

As well as the TCR, functionally equivalent molecules to the TCR are included in the invention. These include any molecule which is functionally equivalent to a TCR which can perform the same function as a TCR. In particular, such molecules include genetically engineered three-domain single-chain TCRs as made by the method described by Chung et al (1994) Proc. Natl. Acad. Sci. USA 91, 12654-12658, and referred to above.

Typically, the TCR or a functionally equivalent molecule to the TCR, recognizes a human Class II HLA molecule expressed on the surface of an antigen-presenting cell and loaded with a peptide according to the first aspect of the invention.

The invention also includes a polynucleotide encoding the TCR or functionally equivalent molecule, and an expression vector encoding the TCR or functionally equivalent molecule thereof. Expression vectors which are suitable for expressing the TCR of the invention include viral vectors such as retroviral vectors, lentiviral vectors, adenoviral vectors, vaccinia vectors (including the replication-deficient MVA strain). It is, however, preferred that the expression vectors are ones which are able to express the TCR in a T cell following transfection.

The invention also includes T cells, preferably CD4⁺ T cells, which have been transfected with a polynucleotide or expression vector which expressed the above mentioned TCR or functionally equivalent molecule. The T cells may be obtained from the patient or, in the case of an allogeneic HSCT patient, from a closely matched donor with respect to HLA type.

The invention also includes a method of combating *Aspergillus* infection in a patient, or of combating allergy to *Aspergillus* in a patient, the method comprising administering to the patient an effective number of *Aspergillus*-specific T cells of the invention, or a population of T cells which have been activated using the peptide or polypeptide of the invention but which have not been selected. For the avoidance of doubt, the *Aspergillus*-specific T cells include those obtainable by the method for producing activated *Aspergillus*-specific T cells *in vitro,* described above, *Aspergillus*-specific CD4⁺ T cells which are CD3⁺ and which recognize the peptide of the invention, in particular the FHT peptide as presented by a HLA-DRB1*04, and those prepared by transfection of a T cell with a polynucleotide or expression vector which expresses the above-mentioned TCR or functionally equivalent molecule. The T cells of the invention are able to combat *Aspergillus* infection selectively by secreting cytokines in response to the antigen. Preferably, the patient is one who carries Class II HLA-DRB1*04.

The T cells may be autologous or they may be HLA matched. If the patient is undergoing allogeneic HSCT, typically the *Aspergillus*-specific T cells are from the donor. When allergy to *Aspergillus* is to be combated in the patient, it is preferred that the T cells are T_{H}1 helper cells or T regulatory cells.

Thus, for patients who demonstrate an allergic reaction to *Aspergillus* antigens, the peptides of the invention may be used to select antigen-specific T regulatory cells which may suppress allergic responses. It will be appreciated the T regulatory cells can be identified by specific markers such as CD25, Foxp3, GITR, and CD127 and the coexpression of these markers with antigen-specificity for the peptides of the invention will allow the selection of T regulatory cells to modulate allergic responses to *Aspergillus* antigens. Thus, a further aspect of the invention provides a method of selecting T regulatory cells for the suppression of an allergic response to *Aspergillus,* the method comprising the use of the peptides of the invention in combination with markers for T regulatory cells in order to create a cellular formulation which can be used to suppress allergic reactions to *Aspergillus.* Conveniently, the cell formulation may be made using high levels of IL-2, and T regulatory cells can be selected as CD3⁺CD25⁺.

The effective clearance of pathogens from the human body sometimes depends on the appropriate T cell response. T_{H}1 responses are associated with cellular pathways involving cytotoxic and phagocytic effector functions while T_{H}2 responses typically involve the production of antibodies by B cells which are "helped" by T_{H}2 T cells. Clearance of *Aspergillus* infections is normally associated with T_{H}1 responses, and the *ex vivo* selection and expansion of antigen-specific T_{H}1 T cells using the peptides of the invention with subsequent infusion of the expanded T_{H}1 T cells into the patient may should facilitate the clearance of the *Aspergillus* infection by reshaping the dominant immune response in the patient.

The peptides of the invention, alone or in combination with antigens from other pathogen, may be used to activate immune cells within a blood or tissue sample or a cellular derivative thereof obtained from the patient or a donor without significant further selection or purification of cell types (an "Unselected Cell Formulation") with a view to infusing the Unselected Cell Formulation in a patient in order to treat or prevent infection by *Aspergillus* whether on a targeted basis or as one of several pathogens which may cause infection in a patient.

Typically, the Unselected Cell Formulation is characterised using the peptide of the invention complexed with a soluble MHC complex (HLA-DRB1*04).

A still further aspect of the invention provides a method of combating *Aspergillus* infection, the method comprising the steps of (1) obtaining T cells from the patient; (2) introducing into said cells a polynucleotide encoding a TCR, or a functionally equivalent molecule, as defined above; and (3) introducing the cells produced in step (2) into the patient. The transfected T cells are able to help fight off the *Aspergillus,* in particular A. *fumigatus.* Preferably, the patients to be treated carry Class II HLA-DRB1 *04.

A still further aspect of the invention provides a method of combating *Aspergillus* infection in a patient, the method comprising the steps of (1) obtaining dendritic cells or other antigen-presenting cells from said patient; (2) contacting said dendritic or other antigen-presenting cells with a peptide or polypeptide or polynucleotide or expression vector of the invention *ex vivo;* and (3) reintroducing the so treated dendritic or other antigen-presenting cells into the patient. Preferably, the patients to be treated carry Class II HLA-DRB1*04.

The methods of combating *Aspergillus* infection, and the pharmaceutical compositions and medicaments, of the invention may be combined with other anti-fungal treatments, such as the use of voriconazole.

Particularly preferred groups of patients to treat include (a) patients who are immunosuppressed following allogeneic HSCT, who may be treated with *Aspergillus-*specific T cells of the invention selected from the donor of the HSCs; (b) patients who are immunosuppressed due to chemotherapy, HIV infection or by the use of immunosuppressive drugs for autoimmune disease, who may be treated with *Aspergillus-specific* T cells of the invention selected from the patient (because these are likely to be present in very small numbers, the cell numbers may be expanded on an *ex vivo* basis; (c) allergy patients in which antigen-specific regulatory T cells are used or T_{H}1 cells are infused to re-direct an T_{H}2 response. In all cases it is preferred that the patient and the donor have the HLA-DRB1*04 allele.

The peptides of the invention may be used to create a monoclonal or polyclonal antibody either on a patient-specific basis or batch manufacturing basis wherein the antibody is used to prevent or treat infection by *Aspergillus* or to induce a primary or secondary humoral or cellular immune response to *Aspergillus* in a patient. The antibody will include idiomatic derivations of antibodies specific for the FHT Peptide, FHTYTIDWTKDAVTW. Preferably, the antibody recognises the peptides of the invention when presented by a Class II HL4-DRB1*04 molecule.

Thus, a further aspect of the invention provides a method of preparing an antibody which recognises the peptide FHTYTIDWTKDAVTW. The method may involve immunisation of an animal such as rabbit or mouse or horse or camel. The method may use hybridoma technology to produce monoclonal antibodies. The method may use *in vitro* selection techniques such as phage display. Preferably, the peptide is presented by a Class II HLA-DRB1*04 molecule.

The antibody may be packaged and presented for use in medicine, particularly for use in combating *Aspergillus* infection. The invention also provides a method of combating *Aspergillus* infection in a patient by administering an effective amount of the antibody to the patient. Preferably, the patient is one who carries Class II HLA-DRB1*04.

The antibody may be monoclonal or polyclonal. Methods of preparing antibodies for administration, including pharmaceutical compositions, are known.

The peptides of the invention may be used within a diagnostic strategy to determine whether a patient may be infected by *Aspergillus* as evidenced by the patient's immune system demonstrating a diagnostically relevant response to the peptides of the invention, typically when presented by HLA-DRB1*04, or to determine whether a patient may be vulnerable to infection by *Aspergillus* by a defective or insufficient response to the peptides of the invention in particular the FHT peptide. The diagnostic applications include the use of the peptides of the invention, typically when presented by HLA-DRB1*04, to identify suitable donors for allogeneic haematopoietic stem cell transplants wherein the objective is to have a donor which demonstrates immunity to *Aspergillus.* The number of responder cells may be very low, so a small scale cell sort may be required to determine this. Thus, the invention includes a method of determining whether an individual is infected with *Aspergillus,* the method comprising determining whether an individual contains any *Aspergillus*-specific T cells, which T cells are able to bind and be activated by a Class II HLA molecule, in particular HLA-DRB1*04 presenting a peptide of the invention. Such T cells can be identified and quantified, for example using the T cell selection systems discussed above. Individuals who contain the *Aspergillus*-specific T cells are likely to be combating an infection, whereas individuals who do not contain, or contain only a low level, may benefit from adoptive immunotherapy according to the invention. On the other hand, individuals who are identified as containing the *Aspergillus*-specific T cells may be useful as donors.

The *Aspergillus-specific* TCR molecules of the invention may be used within a diagnostic strategy to determine whether a patient may be infected by *Aspergillus* as evidenced by the labelling of *Aspergillus*-specific T cells with soluble standalone *Aspergillus*-specific TCR molecules attached to an appropriate labelling system (typically an antibody attached to a fluorochrome). Thus, the invention includes a method of determining whether an individual is infected with *Aspergillus,* the method comprising using a TCR molecule of the invention. The soluble TCR molecules are, in some respects, similar to monoclonal antibodies and can be used to quantify antigen-presenting cells which are presenting a peptide of the invention.

Suitable samples from the patient include a sample following bronchoscopic examination, such as one obtained following lung lavage.

The patients treated by the therapeutic methods of the invention are preferably human patients. The patients treated by the therapeutic methods of the invention preferably are HLA-DRB1*04. Thus, it may be convenient to determine whether an individual to be treated is HLA-DRB1*04 or generally determine the HLA genotype. It is also preferred that the donor of any T cell for use in the therapeutic methods of the invention is HLA-DRB1*04.

The invention will now be described in more detail by reference to the following nonlimiting examples and figures.

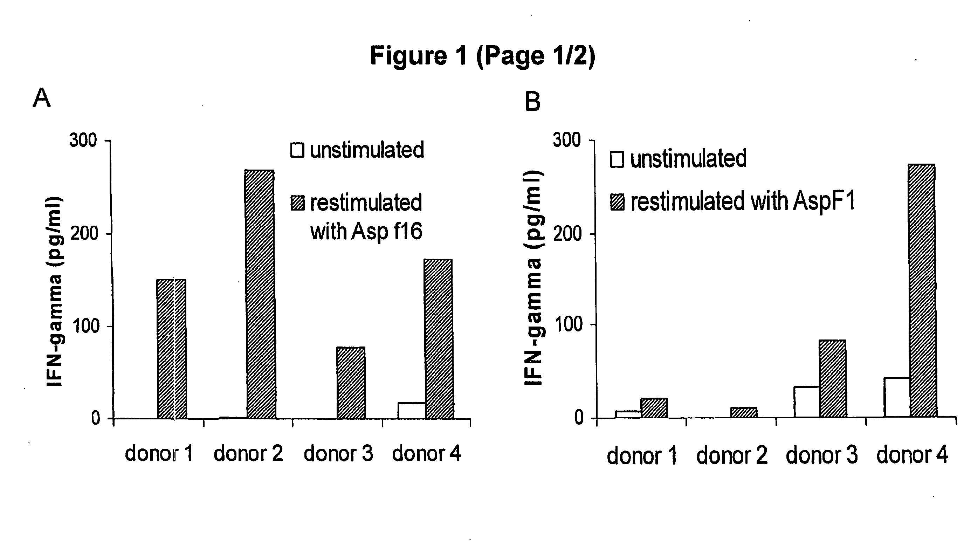

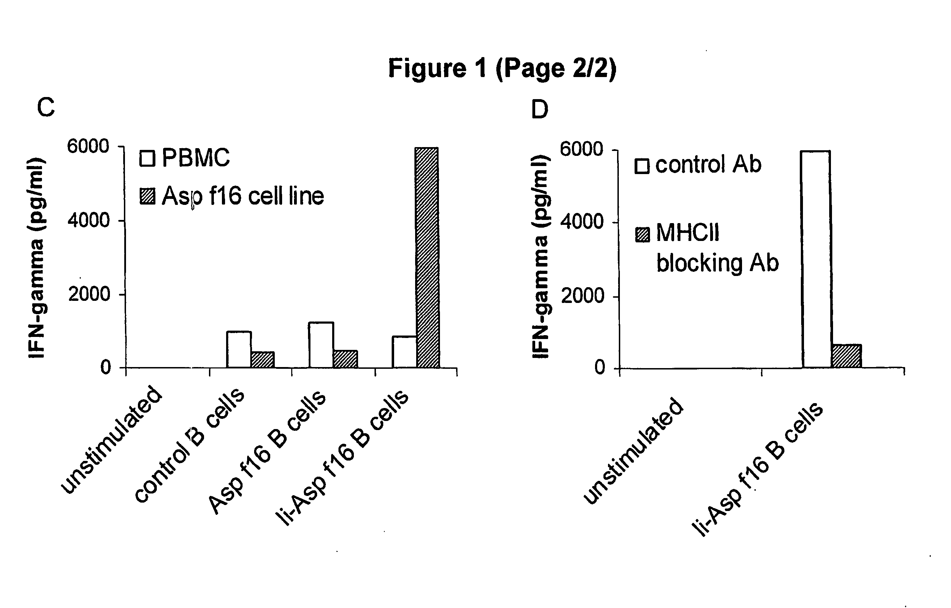

**Figure 1****. Healthy individuals possess functional CD4⁺ memory cells specific for the *A. fumigatus* protein Asp f16.** (A) Restimulation of PBMC from 4 different healthy donors stimulated with recombinant Asp f16 protein for 1 week. (B) Restimulation of PBMC from 4 different healthy donors stimulated with recombinant Asp f1-H136L protein for 1 week. (C) Restimulation of a T cell line generated by stimulation with Asp f16 recombinant protein with autologous B cells transduced with the *aspf16* gene or an invariant chain-asp f16 fusion-gene (*li*-*aspf16*)(representative example). (D) Inhibition of antigen presentation via MHC class II by pre-incubation of antigen-presenting cells with MHC class II blocking antibodies (representative example).

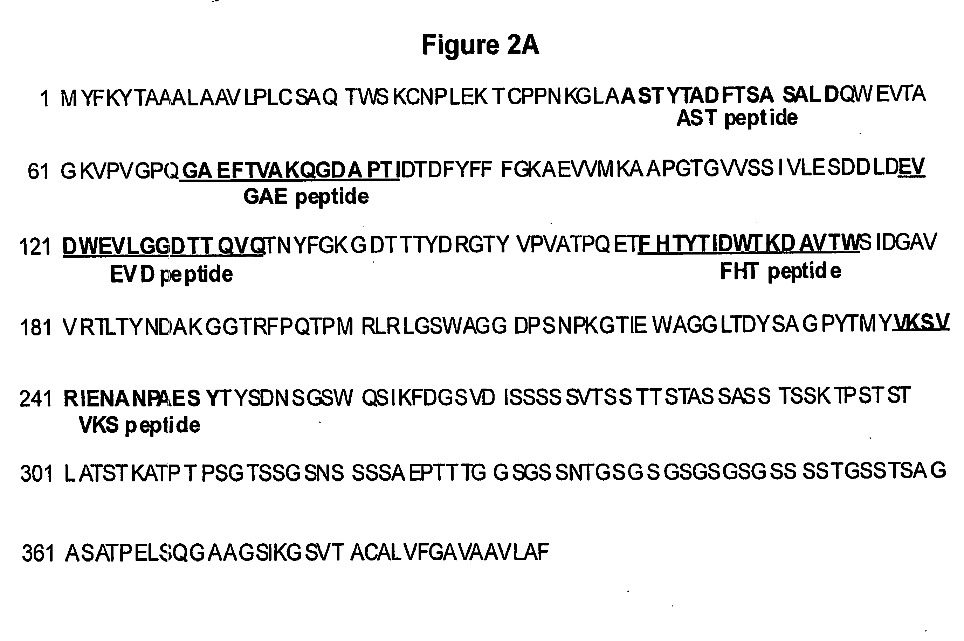

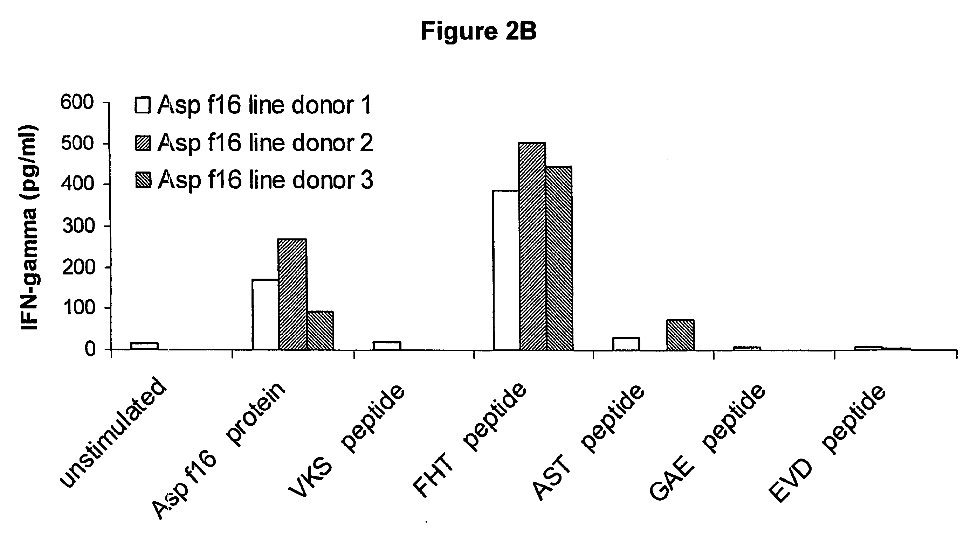

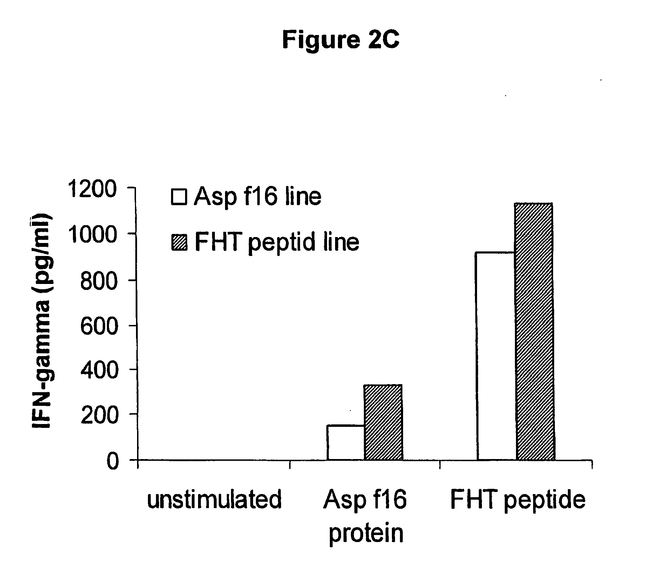

**Figure 2****. Identification of a dominant immunogenic epitope of the Asp f16 protein.** (A) Amino acid sequence of Asp f16 protein and synthetic peptides. (B) Restimulation of 3 Asp f16 recombinant protein-specific cell lines derived from different HLA-DRB1*04-positive healthy donors with 5 different synthetic peptides. (C) T cell lines generated with Asp f16 recombinant protein or FHT peptide restimulated with recombinant protein and peptide (representative example).

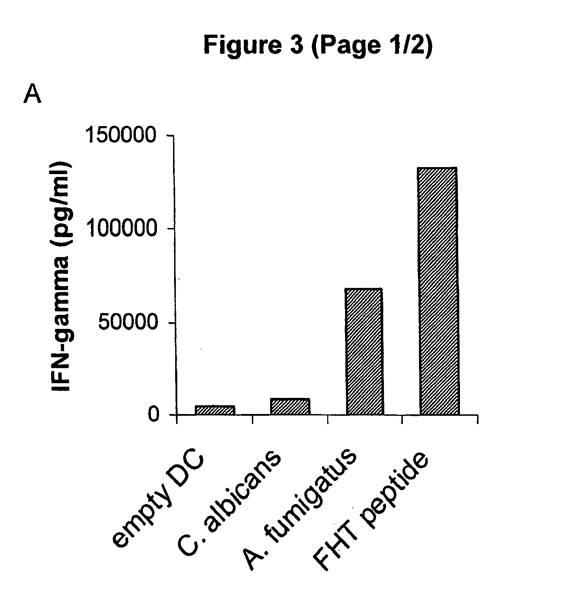

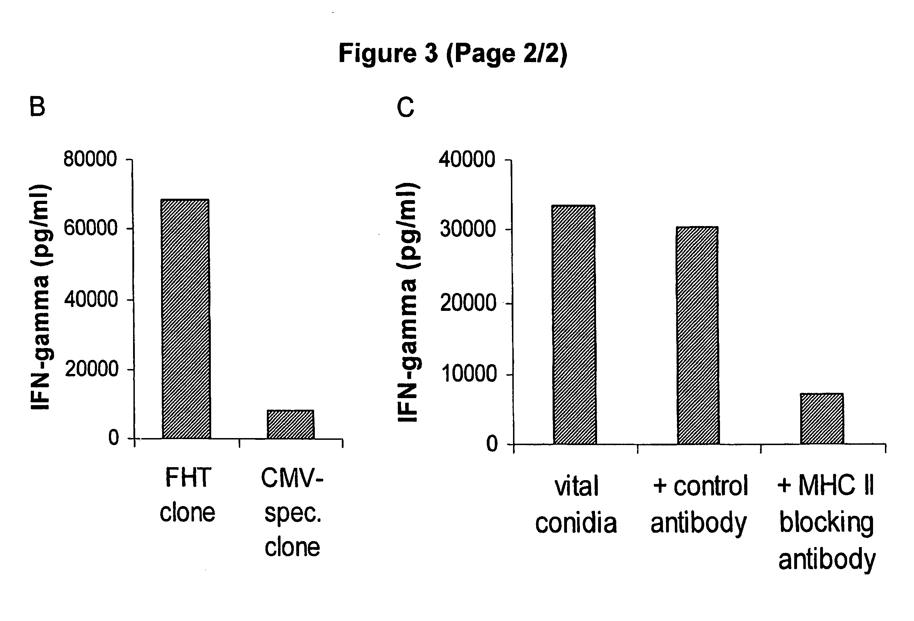

**Figure 3****. Asp f16-specific T cell clones specifically recognize their target antigen on dendritic cells incubated with *A. fumigatus.*** (A) Restimulation of T cell clones specific for the Asp f16 FHT epitope with empty dendritic cells, dendritic cells incubated with either vital *C*. *albicans* or *A*. *fumigatus* conidia or FHT peptide (representative examples). (B) Restimulation of an Asp f16 FHT-specific or CMV-specific T cell clone with dendritic cells incubated with vital *A. fumigatus* (representative examples). (C) Restimulation of Asp f16 FHT-specific T cell clones with dendritic cells incubated with vital *A. fumigatus* in the presence of control or MHC class II-blocking antibody.

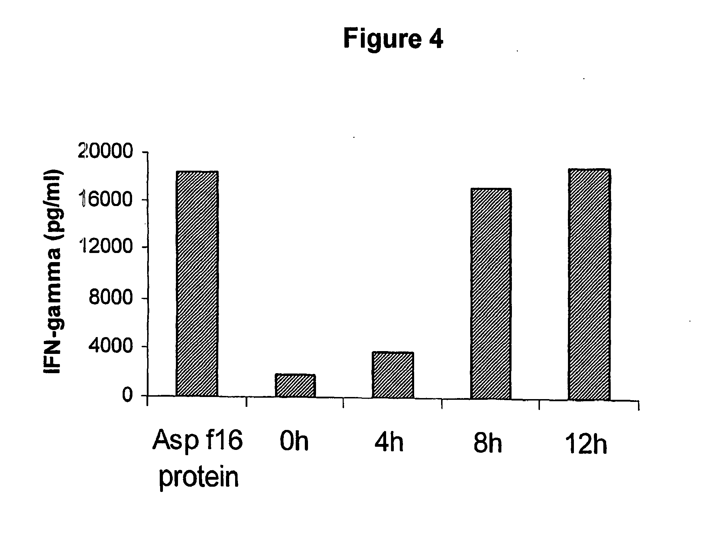

**Figure 4****. The Asp f16-based immunogenicity of *A. fumigatus* strongly increases during germination and hyphal growth.** Restimulation of an Asp f16 FHT-specific T cell clone with dendritic cells incubated with ethanol-killed resting conidia (0h) or fungal preparations killed 4, 8 and 12 hours after germination (representative example).

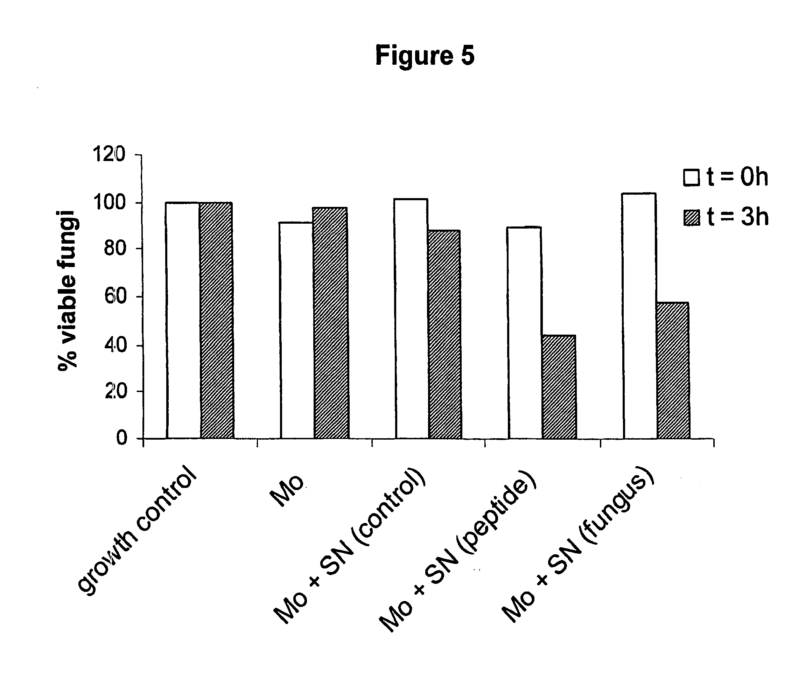

**Figure 5****. Activated Asp f16-specific T cell clones enhance monocyte-mediated fungal killing.** Supernatant from *Aspergillus*-specific T cell clones activated by DC pulsed with peptide or fungal antigen induce fungicidal activity against *Aspergillus* conidia in monocytes. Supernatant from clones co-incubated with unpulsed mature DC or culture medium were used as controls (representative example).

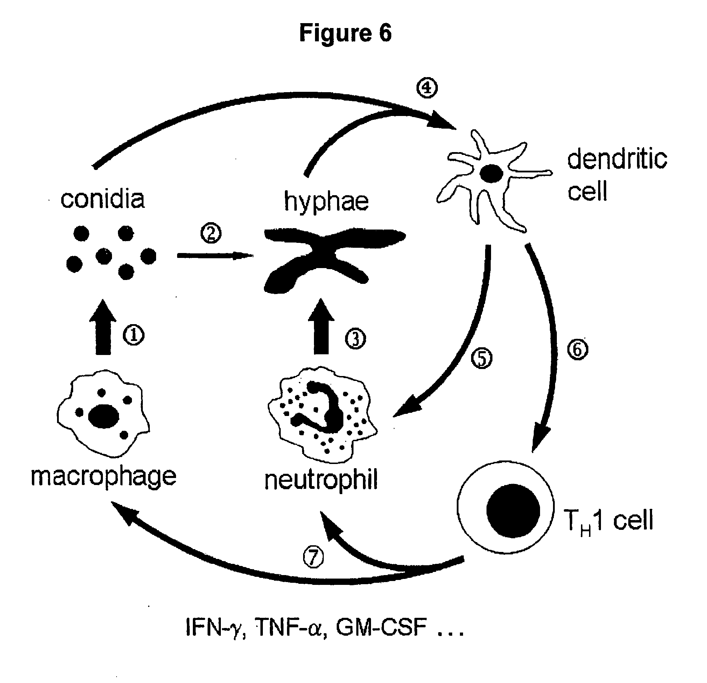

**Figure 6****. Proposed interplay between innate and adaptive immunity to *A. fumigatus.*** Alveolar macrophages phagocytose and kill most of the inhaled conidia ①. Escaping conidia germinate to hyphae ②. which can be destroyed by neutrophilic granulocytes ③. Dendritic cells process fungal antigens ④ and activate innate immune cells ⑤ as well as T_{H}1 cells ⑥ that secrete cytokines to further enhance the antifungal mechanisms of the innate effector cells ⑦ thereby helping to clear the infection.

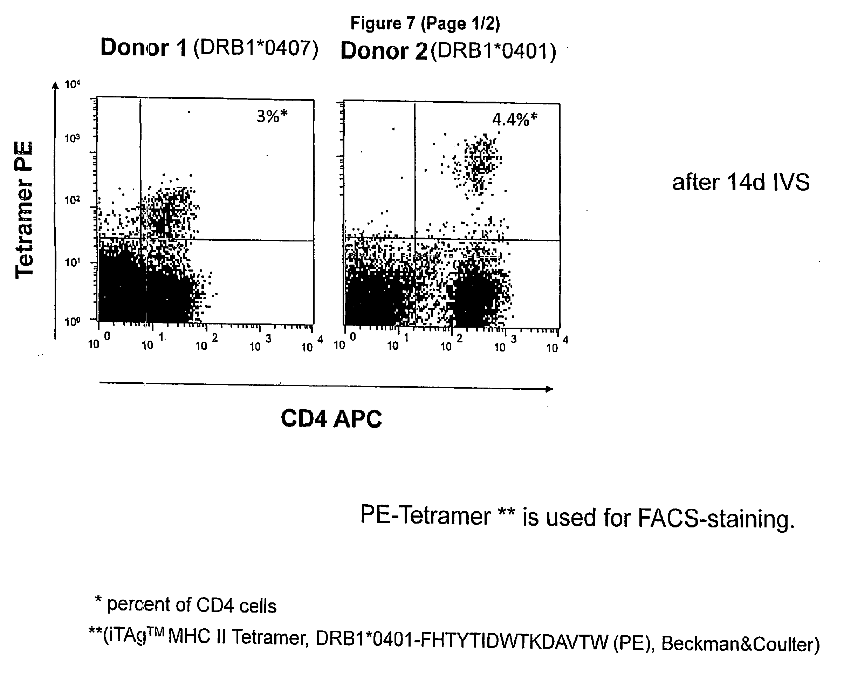

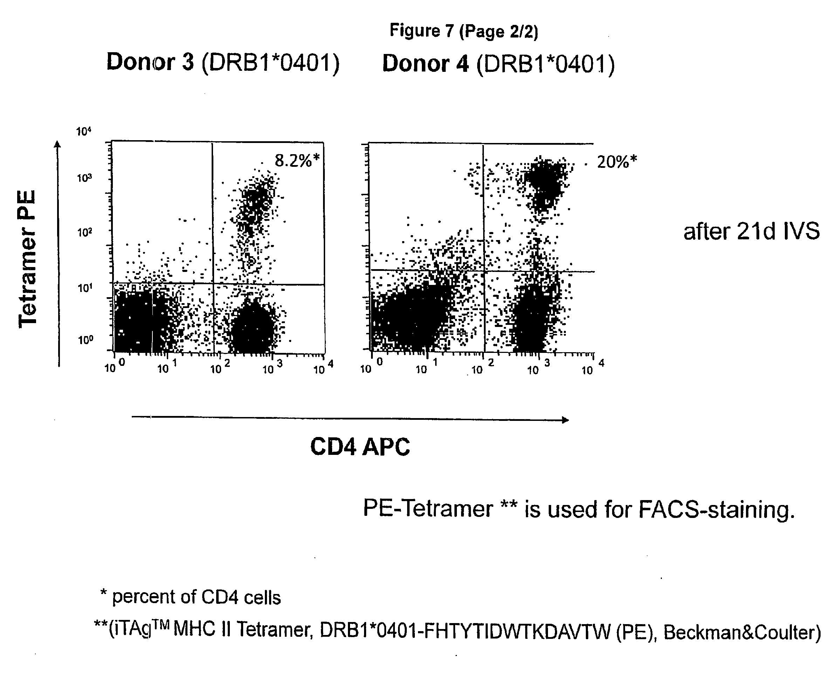

**Figure 7****. Cell lines expanded using the FHT peptide contain T cells that bind specifically to the FHT peptide in the context of DRB1*0401.** PBMC from 3 different donors were expanded with the FHT peptide for 14 or 21 days (IVS - *In vitro* stimulation). Two of the donors were DRB1*0401 positive and one was DRB1*0408 positive. The FHT specific expansions of T cells could be detected using the FHT peptide bound to a multimer of DRB1*0401 showing T cells specific for FHT can be detected by DRB1*0401/FHT multimer staining.

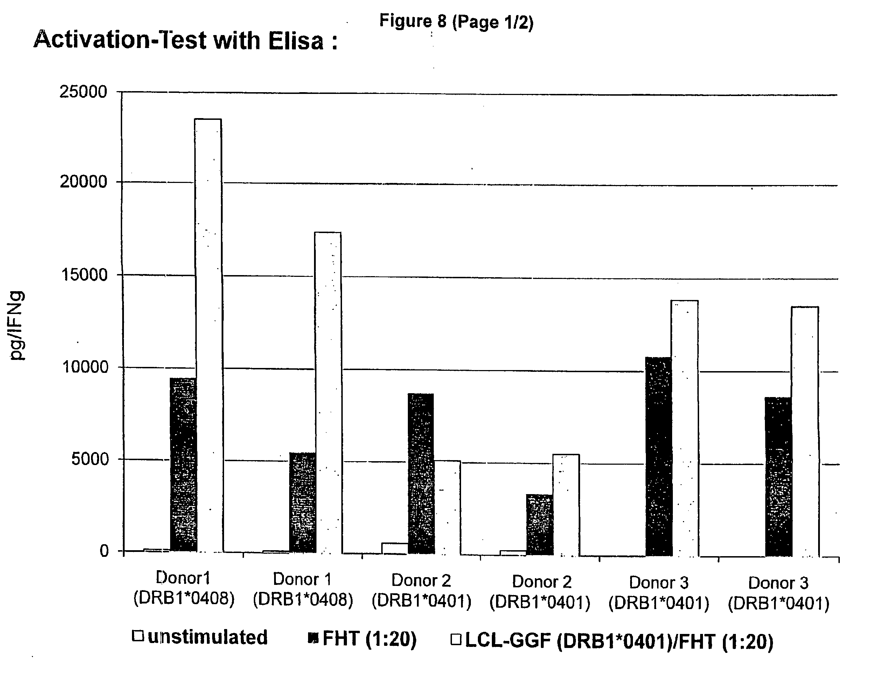

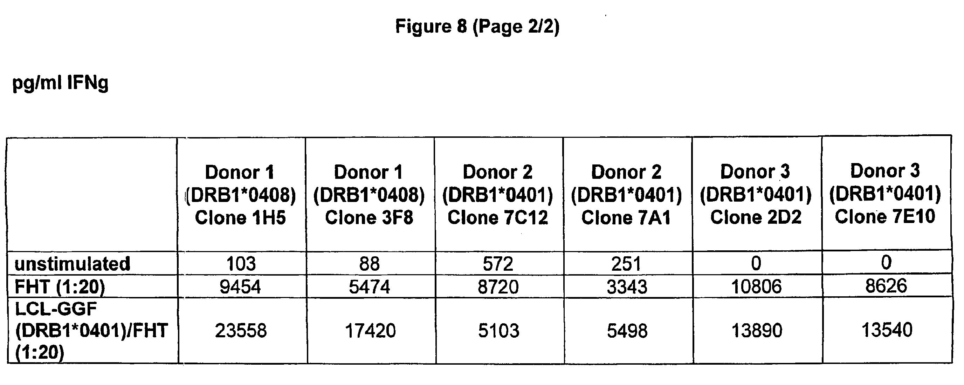

**Figure 8****. T cell clones produce significantly IFNg in response to restimulation with peptide loaded HLA restricted LCL than to peptide alone proving the HLA DRB1*0401/0408 restriction of the FHT peptide.** Using limiting dilution, individual T cell clones were generated. Following expansion, the clones were either left un-stimulated, stimulated with FHT peptide or stimulated with HLA restricted FHT loaded LCL. IFNg production was then measured in a standard ELISA. In all but one of the clones, presenting the FHT peptide in the context of HLA-DRB1*04 greatly increased specific cytokine production compared to peptide alone indicating that FHT specifically binds HLADRB1*04 and the combination of FHT and HLA-DRB1*04 can bind and functionally activate aspergillus specific T cells.

### Example 1: Aspergillus fumigatus and the interaction between Aspergillus fumigatus specific T cells and the innate immune response.

### Summary

Invasive Aspergillosis (IA) caused by the opportunistic pathogenic fungus *Aspergillus fumigatus* constitutes a serious complication especially in patients undergoing allogeneic stem cell transplantation (SCT) and is associated with exceedingly high infection-related morbidity and mortality. In recent years it has become evident that successful disease control is dependent on protective T_{H}1 immune responses critically enhancing the function of innate effector cells primarily responsible for eradication of the pathogen. We evaluated T cell responses to the *A. fumigatus* proteins Asp f1 and Asp f16 in regard to the presence of functional memory cells in potential HSCT donors. The data obtained indicate that most healthy individuals posses Asp f16-specific memory T_{H}1 cells that are able to recognize and can be activated by the pathogen during infection. In contrast Asp f1-specific T cell responses could only be detected in a minority of donors. In addition, an immunodominant epitope for the most abundant MHC class II allele in the Caucasian population was mapped, which elicits T_{H}1 responses in all tested donors. Furthermore, supernatant harvested from activated *Aspergillus-specific* T cells enhanced fungal killing by innate effector cells. The data obtained allows the development of immunotherapeutic protocols for prevention and treatment of *Aspergillus fumigatus* infections.

### Materials and methods

### Cloning, expression and purification of recombinant Asp f16 and Asp f1 protein

The gene encoding the *A. fumigatus* Asp f16 protein (NCBI accession number AY169706) was cloned into the eukaryotic expression vector pcDNA3.1-TOPO (Invitrogen, Karlsruhe, Germany) with N- and C-terminal FLAG^{®}-Tag (Sigma-Aldrich, Muenchen, Germany). The gene was amplified from cDNA kindly provided by Utz Reichard (University of Goettingen, Germany). The gene encoding Asp f1 protein (NCBI accession number M83781) was cloned accordingly, omitting the leader sequence (cDNA kindly provided by Prof. Dr. Stefan Stevanović, University of Tuebingen, Germany). To make expression of the cytotoxic Asp f1 protein in eukaryotic cells possible, we introduced the point mutation His136Leu (Asp f1-H136L) which significantly diminished toxicity in the producer cells, based on the findings by Yang and Kenealy¹⁸ and Kao *et al*¹⁹.

pcDNA3.1-aspf16-FLAG and pcDNA3.1-aspf1-H136L-FLAG expression vector was introduced into the transgenic human cell line phoenix GALV²⁰ via calcium phosphate transfection. Parental as well as transfected producer cells were cultivated in DMEM medium (Invitrogen, Karlsruhe, Germany) supplemented with 10% heat-inactivated fetal calf serum (FCS, Biochrom, Berlin, Germany) and 100 U/ml Penicillin-Streptomycin (Invitrogen, Karlsruhe, Germany). 24 hours after transfection transgene expression from the CMV promoter of the expression vector was enhanced by addition of 1 mM sodium butyrate (Sigma-Aldrich. Muenchen, Germany) to the culture medium²¹.

To purify the cytoplasmically expressed recombinant protein, producer cells were lysed with buffer containing 50 mM Tris-HCl pH 7.4, 150 mM NaCl, 1 mM EDTA and 1% Triton X-100 supplemented with protease inhibitor cocktail (Roche, Mannheim, Germany) and the FLAG-tagged recombinant protein purified under native conditions using M2-agarose according to the manufacturers instructions (Sigma-Aldrich, Muenchen, Germany). Purity of the protein preparation was assessed by coomassie staining of polyacrylamide gels. The synthetic peptides derived from the Asp f16 sequence were kindly provided by Prof. Dr. Stefan Stevanović (University of Tuebingen, Germany).

### Generation of antigen-specific T cell lines and T cell clones

Blood was obtained from HLA-DRB1*-typed healthy donors after informed consent. Peripheral blood mononuclear cells (PBMC) were isolated via centrifugation in Biocoll Separating Solution (Biochrom, Berlin, Germany) and either used directly after preparation or cryopreserved for later use. Cells were cultured in RPMI 1640 with L-Glutamine (invitrogen, Karlsruhe, Germany), supplemented with 10% heat-inactivated, pooled human serum and 100 U/ml Penicillin-Streptomycin (Invitrogen, Karlsruhe, Germany).

Antigen-specific T cell lines were generated by incubation of 1×10⁷ whole PBMC per well in 6-well cell culture plates with 5 µg/ml recombinant protein or 1 µg/ml peptide antigen for 7 days. Lymphocyte cultures were supplemented with 5 U/ml IL-2 (Proleukin, Chiron, Ratingen, Germany) every other day and culture medium replenished as needed.

For generation of antigen-specific T cell clones PBMC were stimulated repeatedly with 1 µg/ml FHT peptide once weekly for 4 weeks. Subsequently T cell clones were generated by limiting dilution in 96-well plates and expanded using the rapid expansion protocol as described by Beck et al¹⁴. A CMVₚₚ₆₅-specific CD4⁺ T cell clone used in control experiments was generated accordingly by limiting dilution of T cells stimulated with pp65 peptide antigen (kindly provided by Jan Diekmann).

### Retroviral transduction of B lymphocytes for presentation of endogenously synthesized antigen

The *aspf16* gene and a *li-aspf16* fusion-gene encoding N-terminally for the first membrane-spanning 80 amino acids of the invariant chain (*li*) were cloned into the retroviral vector pLZNGFR-PGK (kindly provided by Stanley Riddell, Fred Hutchinson Cancer Research Center, Seattle, WA) enabling isolation of stably transduced cells by expression of a truncated form of the nerve growth factor receptor as previously described^{22,23}. Infectious virus used for transduction of primary B cells was produced using the retroviral packaging cell line phoenix GALV²⁰.

CD40L-activated B cells were generated and retrovirally transduced as previously described²⁴. Briefly, whole PBMC were cultured on gamma-irradiated human CD40L-transfected NIH 3T3 cells in IMDM medium (Lonza GmbH, Wuppertal, Germany) supplemented with 10% heat-inactivated, pooled human serum, 4 ng/ml IL-4 (R&D systems, Wiesbaden-Nordenstadt, Germany) and 0.7 µg/ml cyclosporine A (Sigma-Aldrich, Muenchen, Germany). The expanding cells were transferred onto freshly prepared feeder cells every 3-4 days. Retroviral transduction was performed 7 days after culture initiation in the presence of 8 µg/ml polybrene (Sigma-Aldrich, Muenchen, Germany) facilitated by spin infection. Stably transduced B cells were isolated 7 days after transduction based on NGFR expression using magnetic cell separation techniques (Miltenyi Biotech, Bergisch Gladbach, Germany). Transduced CD40L-activated B cells were used in a ratio of 1:10 for restimulation of autologous Asp f16-specific cell lines generated by stimulation with recombinant protein.

### Restimulation of antigen-specific T cells with protein, peptide or transduced B cells and IFN-γ ELISA

Autologous monocytes were isolated from whole PBMC by allowing cells to adhere to tissue culture dishes for 2 hours and subsequent extensive washing to remove non-adherent cells. For presentation of protein antigen, monocytes were cultured overnight in the presence of 5 µg/ml recombinant protein and harvested by mechanical abrasion the next day. For presentation of peptide antigen, monocytes were incubated overnight without antigen and mature cells harvested and pulsed with 1 µg/ml peptide antigen for 1 hour. Transduced autologous B cells were used as antigen-presenting cells (APC) after repeated culture on gamma-irradiated human CD40L-transfected feeder cells.

*Aspergillus*-specific T cell lines or clones were restimulated with protein- or peptide-pulsed monocytes or transduced CD40L-activated B cells in a ratio of 5:1 or 10:1, respectively, for 24 hours and sandwich ELISA performed with culture supernatant using anti-IFN-γ antibodies (Endogen, Perbio Science, Bonn, Germany) according to standard protocols. In some experiments APC were pre-incubated with either 25 µg/ml control antibody or MHC class II blocking antibody (No. 555556_{.} BD Biosciences, Heidelberg, Germany) for 1 hour prior to addition of the T cells. Antibody also remained present during the 24 hours co-incubation of T cells with APC.

### Restimulation of antigen-specific T cell clones with live or ethanol-killed fungus

Dendritic cells (DC) were generated from monocytes by addition of 500 U/ml GM-CSF (PromoCell, Heidelberg, Germany) and 700 U/ml IL-4 (R&D systems, Wiesbaden-Nordenstadt, Germany) to the culture medium. Immature dendritic cells were incubated for 24 hours with either live *A. fumigatus* or *C*. *albicans* conidia or fungus killed by treatment with ethanol 0, 4, 8 or 12 hours after germination and subsequently co-incubated with *Aspergillus* peptide-specific T cell clones for further 24 hours. Dendritic cells used for presentation of protein or peptide antigen were induced to mature by addition of 25 ng/ml TNF-α, 5 ng/ml IL-1β, 10 ng/ml IL-6 (all R&D systems, Wiesbaden-Nordenstadt, Germany) and 1 µg/ml PGE₂ (Pharmacia, Ratingen, Germany) prior to antigen-pulsing and co-incubation with antigen-specific T cells. In one experiment the antigen-presenting dendritic cells fed with live *Aspergillus* conidia were incubated with either 25 µg/ml control antibody or MHC class II blocking antibody (No 555556, BD Biosciences, Heidelberg, Germany) for 1 hour prior to addition of the T cells. Antibody also remained present during the 24 hours co-incubation of T cells with antigen-presenting DC. Culture supernatant harvested after 24 hours was used to determine T cell activation by IFN-γ ELISA and to assess fungal killing by monocytes using confrontation assays.

### Quantification of fungal killing by monocytes

Killing of *A. fumigatus* conidia by monocytes was quantified using confrontation assays. Conidia were co-incubated with monocytes with a multiplicity of infection of 1 for 1, 2 and 3 hours. Monocytes had been pre-incubated with supernatant from stimulated or unstimulated T cells or medium for 30 min prior to the experiment. Controls were plated at 0 hours. Each experimental setting was determined in quadruple. At the appropriate time point cells were lysed by adding 2 ml ice-cold H₂O. 50 µl of this suspension were added to 250 µl of phosphate buffered saline and 90 µl of this dilution was plated on Sabouraud agar. Colonies were quantified after incubation at 37°C over night. At least three independent replications with cells from different donors were performed for each experiment.

### Results

### Identification of a protein-specific recall response to Aspergillus fumigatus

It has been demonstrated that memory T_{H}1-biased T cell responses specific for *Aspergillus fumigatus* can be detected in peripheral blood cells from healthy individuals upon stimulation with *Aspergillus*-derived protein extract¹⁴. Potential candidate antigens with immunogenic properties in this preparations include Asp f1, which has shown to be capable of eliciting T_{H}2 responses in patients suffering from hypersensitivity lung disease^{25,26} and Asp f16, which has been shown to stimulate CD4⁺ as well as CD8⁺ responses in blood cell cultures of healthy individuals^{27,28}. Recombinant Asp f1 and Asp f16 proteins were produced in a eukaryotic expression system and purified by standard methods via introduced FLAG-Tags. We choose specifically a mammalian expression system to circumvent unspecific T cell stimulation by bacterial-derived endotoxin. In order to express the ribotoxin Asp f1 efficiently, we mutated the codon coding for the histidine residue at position 136 involved in catalytic activity (Asp f1-H136L, described by Yang and Kenealy¹⁸ and Kao *et al*¹⁹), which nearly abolished cytotoxicity in the producing cell line.

PBMC from 4 different healthy donors were stimulated for 7 days with purified recombinant Asp f1-H136L or Asp f16 protein. The generated T cell lines were then reexposed for 24 hours to both proteins and the amount of secreted IFN-γ quantified in the supernatant by IFN-γ ELISA. The amount of IFN-γ was compared to fresh autologous PBMC. Cytokine levels of at least 50 pg/ml and/or a more than 5-fold IFN-γ increase in restimulated cultures in comparison to background cytokine levels in unstimulated cultures was defined as significant antigen-specific response. As shown in Figure 1A all tested donors displayed significant levels of IFN-γ after one week of stimulation with recombinant Asp f16 protein, whereas only one of 4 donors responded robustly to recombinant Asp f1-H136L protein, the other 3 donors showing only marginal responses (Figure 1B). As Asp f1 elicited only a response in selected donors, we concentrates our efforts on characterizing the interaction between innate and adaptive immunity in regard to the immunodominant response to Asp f16.

To verify if the observed IFN-γ production is antigen-specific and not due to contamination of our protein preparation, *aspf16* cDNA was cloned into the retroviral vector pLZNGFR-PGK. To enhance antigen presentation via the MHC class II pathway the gene segment encoding for the membrane-spanning N-terminal part of the invariant chain (*li*) was fused to *aspf16* cDNA and the fusion-gene *li-aspf16* cloned into the same retroviral vector. Autologous CD40L-activated B cells from selected donors were transduced with supernatant containing virions encoding either for the cytoplasmically expressed Asp f16 protein or the fusion-protein which is channelled into the MHC class II presentation pathway. Transduced B cells were selected based on the expression of NGFR and used as restimulators of Asp f16-specific T cell lines generated by stimulation with recombinant protein. As shown in Figure 1C, only autologous B cells transduced with the *li-aspf16* fusion-gene induced a significant production of IFN-γ in Asp f16-specific T cell lines, whereas both untransduced B cells as well as B cells modified to express Asp f16 cytoplasmically demonstrated no significant IFN-γ production in comparison to fresh autologous PBMC.

To address the question whether the observed T cell response is either mediated by CD4⁺ or CD8⁺ T cells, a MHC class II neutralizing antibody was added. As shown in Figure 1D Asp f16-triggered IFN-γ production could nearly be abrogated in the presence of blocking antibody, whereas a control antibody had no significant influence on Asp f16-triggered cytokine production. Furthermore, co-culture of retrovirally transduced autologous B cells presenting endogenously synthesized Asp f16 antigen with preselected CD4⁺ or CD8⁺ T lymphocyte subsets did induce only very limited IFN-γ production in the CD8⁺ fraction, whereas CD4⁺ cells showed robust activation of *Aspergillus*-specific memory cells (data not shown).

### Identification of a dominant immunogenic epitope of the Asp f16 protein

In order to define a common MHC class II epitope of the Asp f16 protein, lymphocyte donors were screened for the expression of the most abundant MHC class II allele HLA-DRB1*04 and Asp f16-specific T cell lines generated as described. Peptides were designed based on the SYFPEITHI epitope prediction algorithm²⁹ and synthesized to greater that 80% purity. Asp f16-specific T cell lines of 3 different healthy HLA-DRB1*04-positive donors were exposed to peptide-pulsed autologous monocytes as depicted in Figure 2A. Restimulation of the T cell lines of all donors showed an abundant production of IFN-γ in response to the peptide FHT, suggesting this epitope to be highly immunogenic (Figure 2B). Minor responses were seen with the AST peptide only in 2 out of 3 donors and minimal response to the remaining 3 peptides VKS, GAE and EVD (Figure 2B). To confirm this results a series of criss-cross experiments was performed, where PBMC from HLA-DRB1*04-positive donors were stimulated for one week with either the FHT peptide or the recombinant Asp f16 protein. All T cell cultures were then exposed to both peptide and recombinant protein for 24 hours and IFN-γ production quantified. Again, specific cytokine production could be detected regardless if T cell lines were generated with peptide or protein antigen (Figure 2C). In general higher cytokine levels were seen with cultures stimulated initially with peptide than with recombinant protein.

### Asp f16 epitope-specific T cell clones recognize their target antigen on dendritic cells incubated with live A. fumigatus

The previous results.so far demonstrated that all tested healthy individuals possess Asp f16-specific CD4⁺ memory T_{H}1 cells and that HLA-DRB1*04-positive donors in general exhibit substantial responses to the Asp f16 FHT peptide. As pathogenic fungi, in comparison to viral or bacterial pathogens, are highly complex organisms with manifold and intricate antigenic properties, it is also of interest to determine whether the specific antigen investigated is efficiently processed and presented by fungus-pulsed APC to stimulate T cell activation. To investigate whether *A. fumigatus* epitope-specific T cells can be specifically activated by live fungus, FHT-specific CD4⁺ T cell clones were established by limiting dilution, expanded and restimulated with autologous dendritic cells co-incubated with either vital *Aspergillus fumigatus,* vital *Candida albicans* or with the FHT peptide. As shown in Figure 3A FHT-specific CD4⁺ T cell clones produced high amounts of IFN-γ in response to DC fed with live *Aspergillus* conidia or synthetic peptide, whereas DC fed with live *Candida* failed to induce significant IFN-γ production in FHT-specific CD4⁺ T cells. In a second set of experiments we addressed the question, if dendritic cells exposed to live *Aspergillus* could potentially activate T cells unspecifically. To answer this question a CMVₚₚ₆₅-specific CD4⁺ T-cell clone was co-cultured with autologous DC fed with live *Aspergillus* conidia. As shown in Figure 3B considerably lower IFN-γ levels could be detected in cultures of T cell clones with unrelated specificity in comparison to FHT-specific CD4⁺ T cell clones that showed robust production of cytokines. Furthermore, IFN-γ secretion by the CD4⁺ T cell clone was greatly diminished if the antigen-presenting dendritic cells were pre-treated with MHC class II blocking antibody, as would be expected (Figure 3C).

As *Aspergillus fumigatus* enters its host in the form of airborne conidia and starts invasive infection by initiation of germination and hyphal outgrowth, we investigated whether FHT-specific CD4⁺ T cells can be activated either by conidia, germinating conidia or hyphae.

Different growth stages of *A. fumigatus* were ethanol-killed at defined time points, fed to autologous DC and FHT-specific CD4⁺ T cells added. As depicted in Figure 4 only marginal cytokine production was detected in cultures when ethanol-killed *Aspergillus* conidia were used as antigen. Increasing amounts of cytokine could be detected in a time-dependent fashion in supernatant collected from cultures in which *A. fumigatus* was allowed to germinate and grow before ethanol-killing. In conclusion, FHT-specific CD4⁺ T cells are specifically activated by germinating *A. fumigatus* conidia and hyphae, whereas resting conidia have only limited stimulatory capacity and other fungal pathogens like *C. albicans* fail to induce activation.

### Activated Asp f16-specific T cell clones enhance fungal killing by monocytes

To clarify if the Asp f16-specific T cell clones are actually able to improve the effector function of innate effector cells, monocytes pre-incubated with supernatant taken from T cell cultures activated with FHT peptide-pulsed or fungus-pulsed dendritic cells were co-incubated with vital *A*. *fumigatus* conidia. After 3 hours of incubation monocytes were lysed and the lysate plated on Sabouraud agar to determine the number of viable conidia surviving inside the phagocytes (Figure 5). Whereas control supernatant taken from T cell cultures co-incubated with unpulsed DC had hardly any effect on the killing capacity of monocytes, supernatant taken from T cells activated with FHT peptide-pulsed or fungus-pulsed APC was able to significantly reduce fungal survival after phagocytosis by monocytes, indicating that factors secreted by the activated T cell clones possess the potential to enhance the antifungal mechanisms of monocytes.

### Discussion

In this work we have shown that all tested healthy donors possess memory cells specific for the *A. fumigatus* protein Asp f16, whereas recombinant Asp f1 induces distinct T cell responses only in selected donors. The observed responses were mediated by antigen-specific memory cells of the CD4⁺ T_{H}1 helper cell type responding with IFN-γ secretion upon activation. Conditions biasing T_{H}1 CD4⁺ T cell responses were specifically chosen as it has been shown in the past that healthy in contrast to affected individuals show functional T_{H}1 responses to *A. fumigatus*^{15,17} and only T_{H}1 responses are protective against *Aspergillus* infection in mice^{9,11,13}. Furthermore adoptive transfer of functional T_{H}1 cells is able to control *Aspergillus*-related mortality in transplant recipients in mouse models¹² as well as a human study¹⁶, whereas T_{H}2 responses are often detrimental for the outcome of invasive disease^{8,11,30}. In addition, T_{H}2 biased *Aspergillus-specific* T cell responses are associated with severe atoptic manifestations including allergic bronchopulmonary aspergillosis (ABPA)^{25,31} as well as the late onset of pulmonary Aspergillosis in patients after allogeneic SCT.

Activation of CD8⁺ cells could not be detected upon stimulation either with protein or peptide antigen, which may be due to the relative inefficiency of cross presentation as indicated by other groups^{32,33}. To address the apparent absence of CD8⁺ memory responses more specifically, we established artificial APC by genetically modifying autologous B cells to present the Asp f16 antigen, as this artificial system in general is highly potent in stimulating CD8⁺ T cell responses²³. Despite this approach to augment MHC class I-mediated T cell stimulation we could detect only very limited CD8⁺ recall responses to Asp f16. In contrast, by utilizing the same APC modified to express a MHC class II-targeted li-Asp f16 fusion construct CD4⁺ recall responses were readily detected, confirming appropriate stimulation conditions in our cultures.

The fact that rechallenge with Asp f16, but not Asp f1 induced memory T_{H}1 responses in the majority of tested donors are in accordance with previous data associating the Asp f16 antigen mainly with T_{H}1 responses, whereas the Asp f1 antigen is connected predominantly with T_{H}2 responses and atopic disease. Challenge with the Asp f16 antigen together with CpG deoxyoligonucleotides as adjuvant for instance induces a protective T_{H}1 immune response in mice⁹. Stimulation of human peripheral blood cells with Asp f16 on the other hand, has been shown to induce proliferation and activation of memory cells of different T lymphocyte subtypes, depending on whether blood was taken from healthy individuals or patients suffering from ABPA^{27,34}. The Aspergillus antigen Asp f1 in contrast, has so far been described only in connection with T_{H}2-biased allergic immune responses in mice and humans^{25,26,35}, therefore it is not surprising that T_{H}1 responses to this antigen were observed only occasionally in the healthy individuals investigated in this study.

We were furthermore able to determine one highly immunogenic, HLA-DRB1*04-restricted epitope of the Asp f16 protein, that reproducibly elicited antigen-specific IFN-γ production in all HLA-DRB1*04-positive donors positively tested for responses to the recombinant protein. Ramadan *et al* have already previously described the proliferation and activation of peripheral blood lymphocytes from healthy donors in response to challenge with Asp f16 protein and synthetic peptides^{27,28}; however, the responding cells turned out to be cytolytic CD8⁺ as well as CD4⁺ lymphocytes and the contribution of those cytotoxic cells in host defence against *A. fumigatus* has not been clearly established to date. Although activated CD4⁺ and CD8⁺ T cells as well as NK cells have indeed demonstrated direct antifungal activity against *Cryptococcus neoformans* and *Candida albicans* depending on intimate cell to cell contact^{14,28,36}, the receptors and cognate ligands involved in recognition and the exact mechanism of action remain largely undefined. Besides, cytotoxic T lymphocytes obviously play rather a supportive role in actual control of *A. fumigatus in vivo,* as can be seen in mice selectively depleted of neutrophil function by antibody treatment³⁷. Although the treated animals are exclusively compromised in neutrophil function and still possess a functional T cell repertoire, they quickly succumb to infection with sublethal doses of *A. fumigatus,* whereas untreated wild type animals are able to clear the infection. This observation clearly stresses the pivotal role of innate effector cells in eradication of fungal infections that can be critically enhanced but not made redundant by antigen-specific T lymphocytes.

We have also demonstrated that the generated Asp f16-specific CD4⁺ T_{H}1 T cell clones are specifically and exclusively activated by APC presenting *Aspergillus*-derived Asp f16 antigen either in the form of synthetic peptide, purified protein or viable fungus and are not cross-reactive to APC pulsed with *C. albicans* or purified Asp f1 protein. Also of great interest is the observation, that a CD4⁺ T_{H}1 T cell clone with unrelated specificity responded only with comparably weak cytokine secretion upon contact with DC exposed to live *A. fumigatus,* indicating that the observed stimulatory effect is not attributable to unspecific T cell activation due to the pro-inflammatory milieu generated by dendritic cells after exposure to live fungus. Another fact stressing the antigen-specificity of the response is that T lymphocyte activation is MHC-restricted, as pre-treatment of the antigen-presenting cells with MHC class II blocking antibody strongly impairs IFN-γ production.

One further important factor concerning immunity to *A. fumigatus* is the phenotypic switching from conidia to hyphae during germination, which may help the pathogen to evade host defence³⁸. The change from one morphotype to another is accompanied by loss or change of pathogen-specific signals that are important for the activation and education of antigen-presenting cells² as well as determining the availability of fungal antigens during different growth phases of the pathogen. The *A. fumigatus* Asp f16 FHT peptide-specific T cell clones were able to recognize their target antigen with high specificity and sensitivity on dendritic cells presenting antigen of phagocytosed germinating conidia or outgrown hyphae, whereas only marginal responses were detected after phagocytosis of resting conidia, suggesting that protein expression of the Asp f16 antigen strongly increases during germination.

Furthermore, supernatant taken from T cell cultures co-incubated with fungus-pulsed dendritic cells was able to greatly improve the antifungal activity of monocytes, one of the effector arms of innate immunity against *Aspergillus* infection. The stimulatory effect exerted by activated antigen-specific T_{H}1 cells is probably based on secreted IFN-γ and colony-stimulating factors like G-CSF and GM-CSF, all of which have extensively proven to enhance antifungal effector mechanisms of phagocytes and granulocytes³⁹⁻⁴¹. It has also been shown that IFN-γ together with G-CSF is even able to prevent corticosteroid-induced suppression of monocyte and neutrophil function in vitro^{42,43}, an effect that could potentially also contribute to clearance of invasive Aspergillosis in immunosuppressed transplant recipients. This hypothesis is strengthened by mouse experiments showing that vaccinated animals are protected against subsequent lethal infection with *A. fumigatus,* even when mice are rechallenged during corticosteroid-treatment⁴⁴. A diagram depicting the proposed interplay between innate and adaptive immunity in the control of *A. fumigatus* infection is shown in Figure 6.

With the avenue of immunotherapy being increasingly successful in prevention and treatment of viral infections⁴⁵ we believe that adoptive immunotherapy targeted at *A. fumigatus* will be equally promising. First insight into potential application in the treatment of invasive Aspergillosis in transplant recipients was already obtained by Perruccio *et al*¹⁶, showing that adoptive transfer of *Aspergillus*-specific CD4⁺ T cell clones generated by stimulation with heat-killed conidia was associated with control of *Aspergillus* antigenemia and infection-related mortality in the recipients. Adoptive T cell therapy may be beneficial in patients with evidence of invasive disease, as done in the study mentioned, or as prophylactic or preemptive therapy in asymptomatic transplant recipients. One consideration favouring early preventive therapy could be the risk of overwhelming T cell activation and a resultant excessive cytokine release potentially inducing massive tissue damage if adoptive transfer is conducted in patients with established widespread fungal growth. On the other hand, preemptive T cell therapy highly depends on the quality of available detection assays for the diagnosis of subclinical *Aspergillus* infection. This question is especially of interest as early diagnosis of invasive Aspergillosis still remains difficult, despite the availability of relatively sensitive assays detecting fungal serum galactomannan and β-glucan or polymerase chain reaction-based detection methods⁴⁶. As an alternative to adoptive transfer of T lymphocytes, another strategy is vaccination with dendritic cells presenting *Aspergillus-*derived antigens. This approach may be used either to boost immunity in the stem cell donor prior to stem cell collection, or alternatively, in the patient after allogeneic transplantation⁴⁷.

Taken together these data demonstrate for the first time the characterization of an immunodominant *Aspergillus*-derived antigen and the definition of an epitope specific for an abundant MHC class II allele. Furthermore, CD4⁺ T_{H}1 cells specific for this antigen can be readily activated by DC after uptake of live *Aspergillus,* which in turn are able to condition innate effector cells to enhance control of the fungus. These results provide for immunotherapeutic approaches for prevention and treatment of invasive Aspergillosis in allogeneic HSCT recipients, and other patient groups.

### Example 2: Immune Reconstitution against Aspergillus fumigatus in patients following allogeneic HSCT using Aspergillus-specific T cells selected from the donor using the FHT Peptide

This example is based upon the treatment of patients following allogeneic HSCT. In this application, the donor who has provided the haematopoietic stem cells for transplant is positive for HLA DRB1*04.

The peptide is synthesised on an Applied Biosystems synthesiser, ABI 431A (Foster City, CA, USA) and subsequently purified by HPLC.

Blood is obtained from a HLA-DRB1*-typed donor after informed consent. Donor peripheral blood mononuclear cells (PBMCs) are isolated via centrifugation in Biocoll Separating Solution (Biochrom, Berlin, Germany) and either used directly after preparation or cryopreserved for later use. Cells are cultured in RPMI 1640 with L-Glutamine (Invitrogen, Karlsruhe, Germany), supplemented with 10% heat-inactivated, pooled human serum and 100 U/ml Penicillin-Streptomycin (Invitrogen, Karlsruhe, Germany).

Antigen-specific T cell lines are generated by incubation of 1x10⁷ whole PBMCs per well in 6-well cell culture plates with 1 µg/ml FHT Peptide antigen for 7 days. Lymphocyte cultures were supplemented with 5 U/ml IL-2 (Proleukin, Chiron, Ratingen, Germany) every other day and culture medium replenished as needed. Thereafter, the PBMCs were stimulated repeatedly with 1 µg/ml FHT Peptide once weekly for 4 weeks. Subsequently T cell clones were generated by limiting dilution in 96-well plates and expanded using the rapid expansion protocol as described by Beck et al¹⁴.

Following quality control and potency assays to determine the purity and functional ability of the T cell clones to enhance the innate immune response against *Aspergillus fumigatus* (described earlier under Example 1), the cells are infused into patients following allogeneic HSCT at a dose of 10⁴ *Aspergillus*-specific T cells per kg of patient weight.

Following infusion, the *Aspergillus*-specific T cells will recognise the FHT Peptide presented by antigen-presenting cells present in the patient. The *Aspergillus*-specific T cells will secrete cytokines and other soluble mediators of the human immune response which activate and recruit neutrophils, macrophages, and other cells of the innate immune system to mount a immune response through innate effector functions against the *Aspergillus* pathogen.

### Example 3: Restriction of FHT peptide

### Cell lines expanded using the FHT peptide contain T cells that bind specifically to the FHT peptide in the context of DRB1*0401

PBMC from 3 different donors were expanded with the FHT peptide for 14 or 21 days (IVS - *In vitro* stimulation). Two of the donors were DRB1*0401 positive and one was DRB1*0408 positive. The FHT specific expansions of T cells could be detected using the FHT peptide bound to a multimer of DRB1*0401 showing T cells specific for FHT can be detected by DRB1*0401/FHT multimer staining (see Figure 7).

### T cell clones produce significantly more IFNg in response to re-stimulation with peptide loaded HLA restricted LCL than to peptide alone proving the HLA DRB1*0401/0408 restriction of the FHT peptide

Using limiting dilution, individual T cell clones were generated. Following expansion, the clones were either left un-stimulated, stimulated with FHT peptide or stimulated with HLA restricted FHT loaded LCL. IFNg production was then measured in a standard ELISA. In all but one of the clones, presenting the FHT peptide in the context of HLA-DRB1*04 greatly increased specific cytokine production compared to peptide alone indicating that FHT specifically binds HLADRB1*04 and the combination of FHT and HLA-DRB1*04 can bind and functionally activate aspergillus specific T cells (see Figure 8).

### References

1. Latge JP. The pathobiology of Aspergillus fumigatus. Trends Microbiol. (2001) 9: 382-389.
2. Romani L. Immunity to fungal infections. Nat Rev Immunol. (2004) 4: 1-23.
3. Marr KA, Carter RA, Boeckh M, Martin P, Corey L. Invasive aspergillosis in allogeneic stem cell transplant recipients: changes in epidemiology and risk factors. Blood. (2002)100: 4358-4366.
4. Fukuda T, Boeckh M, Carter RA, et al. Risks and outcomes of invasive fungal infections in recipients of allogeneic hematopoietic stem cell transplants after nonmyeloablative conditioning. Blood. (2003)102: 827-833.
5. Latge JP. Aspergillus fumigatus and aspergillosis. Clin Microbiol Rev. (1999) 12: 310-350.
6. Schaffner A, Douglas H, Braude A. Selective protection against conidia by mononuclear and against mycelia by polymorphonuclear phagocytes in resistance to Aspergillus. Observations on these two lines of defense in vivo and in vitro with human and mouse phagocytes. J Clin Invest. (1982) 69: 617-631.
7. Philippe B, Ibrahim-Granet O, Prevost MC, et al. Killing of Aspergillus fumigatus by alveolar macrophages is mediated by reactive oxidant intermediates. Infect Immun. (2003) 71: 3034-3042.
8. Cenci E, Perito S, Enssle KH, et al. Th1 and Th2 cytokines in mice with invasive aspergillosis. Infect Immun. (1997) 65: 564-570.
9. Bozza S, Gaziano R, Lipford GB, et al. Vaccination of mice against invasive aspergillosis with recombinant Aspergillus proteins and CpG oligodeoxynucleotides as adjuvants. Microbes Infect. (2002) 4: 1281-1290.
10. Ito JI, Lyons JM, Hong TB, et al. Vaccinations with recombinant variants of Aspergillus fumigatus allergen Asp f 3 protect mice against invasive aspergillosis. Infect Immun. (2006) 74: 5075-5084.
11. Cenci E, Mencacci A, Bacci A, Bistoni F, Kurup VP, Romani L. T cell vaccination in mice with invasive pulmonary aspergillosis. J Immunol. (2000) 165: 381-388.
12. Bozza S, Perruccio K, Montagnoli C, et al. A dendritic cell vaccine against invasive aspergillosis in allogeneic hematopoietic transplantation. Blood. (2003) 102: 3807-3814.
13. Rivera A, Van Epps HL, Hohl TM, Rizzuto G, Pamer EG. Distinct CD4+-T-cell responses to live and heat-inactivated Aspergillus fumigatus conidia. Infect Immun. (2005) 73: 7170-7179.
14. Beck O, Topp MS, Koehl U, et al. Generation of highly purified and functionally active human TH1 cells against Aspergillus fumigatus. Blood. (2006) 107: 2562-2569.
15. Hebart H, Bollinger C, Fisch P, et al. Analysis of T-cell responses to Aspergillus fumigatus antigens in healthy individuals and patients with hematologic malignancies. Blood. (2002) 100: 4521-4528.
16. Perruccio K, Tosti A, Burchielli E, et al. Transferring functional immune responses to pathogens after haploidentical hematopoietic transplantation. Blood. (2005) 106: 4397-4406.
17. Grazziutti ML, Rex JH, Cowart RE, Anaissie EJ, Ford A, Savary CA. Aspergillus fumigatus conidia induce a Th1-type cytokine response. J Infect Dis. (1997) 176: 1579-1583.
18. Yang R, Kenealy WR. Effects of amino-terminal extensions and specific mutations on the activity of restrictocin. J Biol Chern. (1992) 267: 16801-16805.
19. Kao R, Shea JE, Davies J, Holden DW. Probing the active site of mitogillin, a fungal ribotoxin. Mol Microbiol. (1998) 29: 1019-1027.
20. Horn PA, Topp MS, Morris JC, Riddell SR, Kiem HP. Highly efficient gene transfer into baboon marrow repopulating cells using GALV-pseudotype oncoretroviral vectors produced by human packaging cells. Blood. (2002) 100: 3960-3967.
21. Palermo DP, DeGraaf ME, Marotti KR, Rehberg E, Post LE. Production of analytical quantities of recombinant proteins in Chinese hamster ovary cells using sodium butyrate to elevate gene expression. J Biotechnol. (1991) 19: 35-47.
22. Kondo E, Topp MS, Kiem HP, et al. Efficient generation of antigen-specific cytotoxic T cells using retrovirally transduced CD40-activated B cells. J Immunol. (2002) 169: 2164-2171.
23. Adamopoulou E, Diekmann J, Tolosa E, et al. Human CD4+ T cells displaying viral epitopes elicit a functional virus-specific memory CD8+ T cell response. J Immunol. (2007) 178: 5465-5472.
24. Schultze JL, Michalak S, Seamon MJ, et al. CD40-activated human B cells: an alternative source of highly efficient antigen presenting cells to generate autologous antigen-specific T cells for adoptive immunotherapy. J Clin Invest. (1997) 100: 2757-2765.
25. Chauhan B, Knutsen A, Hutcheson PS, Slavin RG, Bellone CJ. T cell subsets, epitope mapping, and HLA-restriction in patients with allergic bronchopulmonary aspergillosis. J Clin Invest. (1996) 97: 2324-2331.
26. Kurup VP, Banerjee B, Murali PS, et al. Immunodominant peptide epitopes of allergen, Asp f 1 from the fungus Aspergillus fumigatus. Peptides. (1998) 19: 1469-1477.
27. Ramadan G, Davies B, Kurup VP, Keever-Taylor CA. Generation of Th1 T cell responses directed to a HLA Class II restricted epitope from the Aspergillus f16 allergen. Clin Exp Immunol. (2005) 139: 257-267.
28. Ramadan G, Davies B, Kurup VP, Keever-Taylor CA. Generation of cytotoxic T cell responses directed to human leucocyte antigen Class I restricted epitopes from the Aspergillus f16 allergen. Clin Exp Immunol. (2005) 140: 81-91.
29. Rammensee H, Bachmann J, Emmerich NP, Bachor OA, Stevanovic S. SYFPEITHI: database for MHC ligands and peptide motifs. Immunogenetics. (1999) 50: 213-219.
30. Cenci E, Mencacci A, Del Sero G, et al. Interleukin-4 causes susceptibility to invasive pulmonary aspergillosis through suppression of protective type I responses. J Infect Dis. (1999) 180: 1957-1968.
31. Kurup VP, Xia JQ, Crameri R, et al. Purified recombinant A. fumigatus allergens induce different responses in mice. Clin Immunol. (2001) 98: 327-336.
32. Harding CV, Song R. Phagocytic processing of exogenous particulate antigens by macrophages for presentation by class I MHC molecules. J Immunol. (1994) 153: 4925-4933.
33. Maecker HT, Ghanekar SA, Suni MA, He XS, Picker LJ, Maino VC. Factors affecting the efficiency of CD8+ T cell cross-priming with exogenous antigens. J Immunol. (2001) 166: 7268-7275.
34. Banerjee B, Kurup VP, Greenberger PA, Johnson BD, Fink JN. Cloning and expression of Aspergillus fumigatus allergen Asp f 16 mediating both humoral and cell-mediated immunity in allergic bronchopulmonary aspergillosis (ABPA). Clin Exp Allergy. (2001) 31: 761-770.
35. Chauhan B, Santiago L, Kirschmann DA, et al. The association of HLA-DR alleles and T cell activation with allergic bronchopulmonary aspergillosis. J Immunol. (1997) 159: 4072-4076.
36. Levitz SM, Mathews HL, Murphy JW. Direct antimicrobial activity of T cells. Immunol Today. (1995) 16:387-391.
37. Cenci E, Mencacci A, Fe d'Ostiani C, et al. Cytokine- and T helper-dependent lung mucosal immunity in mice with invasive pulmonary aspergillosis. J Infect Dis. (1998) 178: 1750-1760.
38. Netea MG, Warris A, Van der Meer JW, et al. Aspergillus fumigatus evades immune recognition during germination through loss of toll-like receptor-4-mediated signal transduction. J Infect Dis. (2003) 188: 320-326.
39. Nemunaitis J. Use of macrophage colony-stimulating factor in the treatment of fungal infections. Clin Infect Dis. (1998) 26: 1279-1281.
40. Roilides E, Uhlig K, Venzon D, Pizzo PA, Walsh TJ. Enhancement of oxidative response and damage caused by human neutrophils to Aspergillus fumigatus hyphae by granulocyte colony-stimulating factor and gamma interferon. Infect Immun. (1993) 61: 1185-1193.
41. Gil-Lamaignere C, Winn RM, Simitsopoulou M, Maloukou A, Walsh TJ, Roilides E. Inteferon gamma and granulocyte-macrophage colony-stimulating factor augment the antifungal activity of human polymorphonuclear leukocytes against Scedosporium spp.: comparison with Aspergillus spp. Med Mycol. (2005) 43: 253-260.
42. Roilides E, Uhlig K, Venzon D, Pizzo PA, Walsh TJ. Prevention of corticosteroid-induced suppression of human polymorphonuclear leukocyte-induced damage of Aspergillus fumigatus hyphae by granulocyte colony-stimulating factor and gamma interferon. Infect Immun. (1993) 61: 4870-4877.
43. Roilides E, Lyman CA, Mertins SD, et al. Ex vivo effects of macrophage colony-stimulating factor on human monocyte activity against fungal and bacterial pathogens. Cytokine. (1996) 8: 42-48.
44. Ito JI, Lyons JM. Vaccination of corticosteroid immunosuppressed mice against invasive pulmonary aspergillosis. J Infect Dis. (2002) 186: 869-871.
45. Einsele H, Kapp M, Grigoleit GU. CMV-specific T cell therapy. Blood Cells Mol Dis. (2008) 40: 71-75.
46. Segal BH, Walsh TJ. Current approaches to diagnosis and treatment of invasive aspergillosis. Am J Respir Crit Care Med. (2006) 173: 707-717.
47. Grigoleit GU, Kapp M, Hebart H, Fick K, Beck R, Jahn G, Einsele H. Dendritic cell vaccination in allogeneic stem cell recipients: Induction of human cytomegalovirus (HCMV)-specific cytotoxic T lymphocyte response even in patients receiving a transplant from an HCMV-seronegative donor. J Infect Dis. (2007) 196: 699-704
48. Zhu F, Ramadan G, Davies B, Margolis DA, Keever-Taylor CA. Stimulation by means of dendritic cells followed by Epstein-Barr virus-transformed B cells as antigen-presenting cells is more efficient than dendritic cells alone in inducing Aspergillus f16-specific cytotoxic T cell responses. Clin. Exp Immunol. 151, 284-296

## Claims

1. A peptide of less than 5000 molecular weight comprising the amino acid sequence FHTYTIDWTKDAVTW wherein the peptide is capable of binding HLA-DRB1*04 and wherein when bound to HLA-DRB1*04 the peptide-bound HLA-DRB1*04 is capable of identifying T cells specific for *Aspergillus fumigatus.*

2. A peptide according to Claim 1 wherein the peptide includes non-peptide bonds.

3. A polypeptide which is a fusion of the peptide of Claim 1 and another peptide, wherein the other peptide is an invariant chain of an HLA molecule and wherein the peptide of claims 1 or 2 is able to occupy the peptide binding groove of the HLA molecule.

4. A complex comprising a Class II HLA-DRB1*04 molecule bound to a peptide, wherein the peptide is a peptide of less than 5000 molecular weight comprising the amino acid sequence FHTYTIDWTKDAVTW or a portion of at least 11 amino acids of the given amino acid sequence , or a variant of the given amino acid sequence wherein the side chains of one or two or three of the amino acid residues are altered, wherein the peptide comprising the portion, or variant, is capable of binding HLA-DRB1*04, and wherein when bound to HLA-DRB1*04 the peptide-bound HLA-DRB1*04 is capable of identifying T cells specific for *Aspergillus fumigatus*

5. A complex according to Claim 4 which is a soluble complex.

6. *In vitro* use of a complex according to Claims 4 or 5 for isolating an *Aspergillus-*specific T cell, or for identifying an *Aspergillus*-specific T cell in a sample.

7. A method for selecting *Aspergillus*-specific T cells, the method comprising contacting a population of T cells with a peptide presented in a Class II HLA-DRB1*04 molecule *in vitro,* wherein the peptide is a peptide as defined in Claim 4.

8. A method according to Claim 7 wherein the Class II HLA molecule is present on the surface of an antigen-presenting cell.

9. A complex according to Claim 4 or a method according to Claim 7 or 8 wherein the Class II HLA molecule is a synthetic soluble Class II HLA molecule.

10. A method according to any one of Claims 8 to 9 wherein the selected *Aspergillus-*specific T cell is isolated.

11. *Aspergillus*-specific T cells which are able to recognise the HLA-DRB1*04-presented peptide as defined in Claim 4.

12. A T-cell receptor (TCR) which detects *Aspergillus* infection, the TCR being obtainable from the *Aspergillus*-specific T cells of Claim 11, wherein the TCR, recognises human class II MHC molecules expressed on the surface of an antigen-presenting cell and loaded with a peptide as defined in Claim 4.

13. A TCR according to Claim 12 which is a three-domain single chain TCR.

14. *Aspergillus-*specific T cells as defined in Claim 11 or T cells comprising a polynucleotide encoding a TCR as defined in Claim 12 or a population of T cells which has been activated using the peptide as defined in Claim 4 for use in medicine, for example for use in combating *Aspergillus* infection, or for use in combating allergy to *Aspergillus,* in a patient, optionally wherein the *Aspergillus*-specific T cells are autologous to the patient, or the T cells are from a donor.

15. A method of assessing the amount of *Aspergillus*-specific T cells in an individual the method comprising contacting a blood sample from the individual with the complex of Claim 4.

16. A polynucleotide encoding a peptide as defined in Claim 1 or a polypeptide according to Claim 3 or a TCR as defined in Claim 12.

17. A pharmaceutical composition comprising a peptide as defined in Claim 1 or a polypeptide according to Claim 3 or a polynucleotide according to Claim 16 or *Aspergillus* specific T cells as defined in Claim 11 and a pharmaceutically acceptable carrier.

18. A peptide as defined in Claim 1 or a polypeptide according to Claim 3 or a polynucleotide according to Claim 16 for use in medicine, preferably wherein the individual to be treated is a human individual who carries Class II HLA-DRB1*04, for example for use in combating *Aspergillus* infection or for combating allergy to *Aspergillus.*

19. A vaccine against *Aspergillus* infection comprising a peptide as defined in Claim 1 or a polypeptide according to Claim 3 or a polynucleotide according to Claim 16.

20. An antigen presenting cell, such as a dendritic cell, which is presenting a peptide as defined in Claim 1 or a polypeptide according to Claim 3, preferably wherein the antigen presenting cell carries Class II HLA-DRB1*04.

21. An antigen presenting cell according to Claim 20 for use in medicine, optionally as a vaccine against *Aspergillus* infection or optionally for use in combating *Aspergillus* infection in a patient, preferably wherein the patient is one who carries Class II HLA-DRB1*04.

22. An antibody which selectively recognises the peptide FHTYTIDWTKDAVTW when presented by Class II HLA-DRB1*04.

23. An antibody according to Claim 22 for use in medicine, such as for use in combating *Aspergillus* infection, preferably wherein the recipient is one who carries Class II HLA-DRB1*04.

24. An *in vitro* method of determining whether an individual is infected with *Aspergillus,* the method comprising determining whether an individual contains *Aspergillus*-specific T cells as defined in Claim 11, preferably wherein the individual is one who carries Class II HLA-DRB1*04.

25. An *in vitro* method of determining whether an individual is infected with *Aspergillus,* the method comprising using a TCR according to Claim 12, preferably wherein the individual is one who carries Class II HLA-DRB1*04.

## Patentansprüche

1. Peptid einer relativen Molekülmasse von weniger als 5000, das die Aminosäuresequenz FHTYTIDWTKDAVTW umfasst, wobei das Peptid zur Bindung an HLA-DRB1*04 fähig ist und wobei, wenn die Bindung an HLA-DRB1*04 erfolgt ist, das Peptid-gebundene HLA-DRB1*04 zur Identifizierung von für *Aspergillus fumigatus* spezifischen T-Zellen fähig ist.

2. Peptid nach Anspruch 1, wobei das Peptid Nichtpeptidbindungen umfasst.

3. Polypeptid, das eine Fusion von dem Peptid nach Anspruch 1 und einem anderen Peptid ist, wobei das andere Peptid eine invariante Kette eines HLA-Moleküls ist und wobei das Peptid nach Anspruch 1 oder 2 die Peptidbindungsgrube des HLA-Moleküls besetzen kann.

4. Komplex, der ein an ein Peptid gebundenes Klasse-II-HLA-DRB1*04-Molekül umfasst, wobei das Peptid ein Peptid einer relativen Molekülmasse von weniger als 5000 ist, das die Aminosäuresequenz FHTYTIDWTKDAVTW oder einen Teil von mindestens 11 Aminosäuren der angegebenen Aminosäuresequenz oder eine Variante der angegebenen Aminosäuresequenz, wobei die Seitenketten von einem oder zwei oder drei der Aminosäurereste verändert sind, umfasst, wobei das den Teil oder eine Variante umfassende Peptid zur Bindung an HLA-DRB1*04 fähig ist und wobei, wenn die Bindung an HLA-DRB1*04 erfolgt ist, das Peptid-gebundene HLA-DRB1*04 zur Identifizierung von für *Aspergillus fumigatus* spezifischen T-Zellen fähig ist.

5. Komplex nach Anspruch 4, wobei dieser ein löslicher Komplex ist.

6. *In vitro*-Verwendung eines Komplexes nach Anspruch 4 oder 5 zur Isolierung einer *Aspergillus*-spezifischen T-Zelle oder zur Identifizierung einer *Aspergillus*-spezifischen T-Zelle in einer Probe.

7. Verfahren zur Selektion von *Aspergillus*-spezifischen T-Zellen, wobei das Verfahren das Inkontaktbringen einer Population von T-Zellen mit einem in einem Klasse-II-HLA-DRB1*04-Molekül präsentierten Peptid *in vitro,* wobei das Peptid ein Peptid gemäß der Definition in Anspruch 4 ist, umfasst.

8. Verfahren nach Anspruch 7, wobei das Klasse-II-HLA-Molekül auf der Oberfläche einer Antigen-präsentierenden Zelle vorhanden ist.

9. Komplex nach Anspruch 4 oder Verfahren nach Anspruch 7 oder 8, wobei das Klasse-II-HLA-Molekül ein synthetisches lösliches Klasse-II-HLA-Molekül ist.

10. Verfahren nach einem der Ansprüche 8 bis 9, wobei die selektierte *Aspergillus*-spezifische T-Zelle isoliert wird.

11. *Aspergillus*-spezifische T-Zellen, die das von HLA-DRB1*04 präsentierte Peptid gemäß der Definition im Anspruch 4 erkennen können.

12. T-Zellen-Rezeptor (TCR), der eine *Aspergillus*-Infektion detektiert, wobei der TCR aus den *Aspergillus*-spezifischen T-Zellen nach Anspruch 11 erhältlich ist, wobei der TCR humane Klasse-II-MHC-Moleküle, die auf der Oberfläche einer Antigen-präsentierenden Zelle exprimiert und mit einem Peptid gemäß der Definition in Anspruch 4 beladen sind, erkennt.

13. TCR nach Anspruch 12, wobei dies ein Drei-Domänen-Einzelketten-TCR ist.

14. *Aspergillus*-spezifische T-Zellen gemäß der Definition in Anspruch 11 oder T-Zellen, die ein Polynucleotid mit Codierung für einen TCR gemäß der Definition in Anspruch 12 umfassen, oder Population von T-Zellen, die unter Verwendung des Peptids gemäß der Definition in Anspruch 4 aktiviert wurde, zur Verwendung in der Medizin, beispielsweise zur Verwendung bei der Bekämpfung einer *Aspergillus*-Infektion oder zur Verwendung bei der Bekämpfung einer Allergie auf *Aspergillus* bei einem Patienten, wobei optional die *Aspergillus*-spezifischen T-Zellen zum Patienten autologe sind oder die T-Zellen von einem Spender stammen.

15. Verfahren zur Bestimmung der Menge von *Aspergillus-*spezifischen T-Zellen in einem Individuum, wobei das Verfahren das Inkontaktbringen einer Blutprobe von dem Individuum mit dem Komplex nach Anspruch 4 umfasst.

16. Polynucleotid mit Codierung für ein Peptid gemäß der Definition in Anspruch 1 oder ein Polypeptid gemäß Anspruch 3 oder einen TCR gemäß der Definition in Anspruch 12.

17. Pharmazeutische Zusammensetzung, die ein Peptid gemäß der Definition in Anspruch 1 oder ein Polypeptid gemäß Anspruch 3 oder ein Polynucleotid gemäß Anspruch 16 oder *Aspergillus*-spezifische T-Zellen gemäß der Definition in Anspruch 11 und einen pharmazeutisch akzeptablen Träger umfasst.

18. Peptid gemäß der Definition in Anspruch 1 oder Polypeptid gemäß Anspruch 3 oder Polynucleotid gemäß Anspruch 16 zur Verwendung in der Medizin, vorzugsweise wenn das zu behandelnde Individuum ein individueller Mensch ist, der Klasse-II-HLA-DRB1*04 trägt, beispielsweise zur Verwendung bei der Bekämpfung einer *Aspergillus*-Infektion oder zur Bekämpfung einer Allergie auf *Aspergillus.*

19. Vakzin gegen eine *Aspergillus*-Infektion, das ein Peptid gemäß der Definition im Anspruch 1 oder ein Polypeptid gemäß Anspruch 3 oder ein Polynucleotid gemäß Anspruch 16 umfasst.

20. Antigen-präsentierende Zelle, beispielsweise dendritische Zelle, die ein Peptid gemäß der Definition in Anspruch 1 oder ein Polypeptid gemäß Anspruch 3 präsentiert, wobei vorzugsweise die Antigen-präsentierende Zelle Klasse-II-HLA-DRB1*04 trägt.

21. Antigen-präsentierende Zelle nach Anspruch 20 zur Verwendung in der Medizin, optional als Vakzin gegen eine *Aspergillus*-Infektion oder optional zur Verwendung bei der Bekämpfung einer *Aspergillus*-Infektion bei einem Patienten, wobei vorzugsweise der Patient einer ist, der Klasse-II-HLA-DRB1*04 trägt.

22. Antikörper, der selektiv das Peptid FHTYTIDWTKDAVTW, wenn es durch Klasse-II-HLA-DRB1*04 präsentiert wird, erkennt.

23. Antikörper nach Anspruch 22 zur Verwendung in der Medizin, beispielsweise zur Verwendung bei der Bekämpfung einer *Aspergillus*-Infektion, wobei vorzugsweise der Empfänger einer ist, der Klasse-II-HLA-DRB1*04 trägt.

24. *In vitro*-Verfahren zur Bestimmung, ob ein Individuum mit *Aspergillus* infiziert ist, wobei das Verfahren das Bestimmen, ob ein Individuum *Aspergillus*-spezifische T-Zellen gemäß der Definition im Anspruch 11 enthält, umfasst, wobei vorzugsweise das Individuum eines ist, das Klasse-II-HLA-DRB1*04 trägt.

25. *In vitro*-Verfahren zur Bestimmung, ob ein Individuum mit *Aspergillus* infiziert ist, wobei das Verfahren die Verwendung eines TCR nach Anspruch 12 umfasst, wobei vorzugsweise das Individuum eines ist, das Klasse-II-HLA-DRB1*04 trägt.

## Revendications

1. Peptide de masse moléculaire inférieure à 5 000 comprenant la séquence d'acides aminés FHTYTIDWTKDAVTW, dans lequel le peptide est capable de se lier à HLA-DRB1*⁰4 et dans lequel une fois lié à HLA-DRB1*04, la HLA-DRB1*04 liée au peptide est capable d'identifier des cellules T spécifiques d'*Aspergillus fumigatus*.

2. Peptide selon la revendication 1, dans lequel le peptide inclut des liaisons non peptidiques.

3. Polypeptide qui est une fusion du peptide de la revendication 1 et d'un autre peptide, dans lequel l'autre peptide est une chaîne invariante d'une molécule HLA et dans lequel le peptide des revendications 1 ou 2 est capable d'occuper le sillon de liaison au peptide de la molécule HLA.

4. Complexe comprenant une molécule HLA-DRB1*04 de classe II liée à un peptide, dans lequel le peptide est un peptide de masse moléculaire inférieure à 5 000 comprenant la séquence d'acides aminés FHTYTIDWTKDAVTW, ou une portion d'au moins 11 acides aminés de la séquence d'acides aminés donnée, ou un variant de la séquence d'acides aminés donnée dans lequel les chaînes latérales d'un ou de deux ou de trois des résidus d'acide aminé sont modifiées, dans lequel le peptide comprenant la portion, ou le variant, est capable de se lier à HLA-DRB1*04 et dans lequel lorsqu'il est lié à HLA-DRB1*04, la HLA-DRB1*04 liée au peptide est capable d'identifier des cellules T spécifiques d'*Aspergillus fumigatus.*

5. Complexe selon la revendication 4, qui est un complexe soluble.

6. Utilisation *in vitro* d'un complexe selon les revendications 4 ou 5, pour isoler une cellule T spécifique *d'Aspergillus,* ou pour identifier une cellule T spécifique *d'Aspergillus* dans un échantillon.

7. Procédé de sélection de cellules T spécifiques *d'Aspergillus,* le procédé comprenant la mise en contact d'une population de cellules T avec un peptide présenté dans une molécule HLA-DRB1*04 de classe II *in vitro,* dans lequel le peptide est un peptide tel que défini dans la revendication 4.

8. Procédé selon la revendication 7, dans lequel la molécule HLA de classe II est présente sur la surface d'une cellule présentant l'antigène.

9. Complexe selon la revendication 4 ou procédé selon la revendication 7 ou 8, dans lequel la molécule HLA de classe II est une molécule HLA de classe II soluble synthétique.

10. Procédé selon l'une quelconque des revendications 8 à 9, dans lequel la cellule T spécifique *d'Aspergillus* sélectionnée est isolée.

11. Cellules T spécifiques d'*Aspergillus* qui sont capables de reconnaître le peptide présenté par HLA-DRB1*04 tel que défini dans la revendication 4.

12. Récepteur de cellule T (RCT) qui détecte une infection par *Aspergillus,* le RCT pouvant être obtenu à partir des cellules T spécifiques *d'Aspergillus* de la revendication 11, dans lequel le RCT reconnaît des molécules de MHC classe II humaines exprimées sur la surface d'une cellule présentant l'antigène et chargées avec un peptide tel que défini dans la revendication 4.

13. RCT selon la revendication 12, qui est un RCT monocaténaire à trois domaines.

14. Cellules T spécifiques d'*Aspergillus* selon la revendication 11, ou cellules T comprenant un polynucléotide codant un RCT tel que défini dans la revendication 12, ou population de cellules T qui ont été activées en utilisant le peptide tel que défini dans la revendication 4, pour utilisation en médecine, par exemple pour utilisation pour combattre une infection par *Aspergillus,* ou pour utilisation pour combattre une allergie à *Aspergillus,* chez un patient, éventuellement dans lesquels les cellules T spécifiques d'*Aspergillus* sont autologues au patient, ou bien les cellules T proviennent d'un donneur.

15. Procédé pour estimer la quantité de cellules T spécifiques d'*Aspergillus* chez un individu, le procédé comprenant la mise en contact d'un échantillon de sang issu de l'individu avec le complexe de la revendication 4.

16. Polynucléotide codant un peptide tel que défini dans la revendication 1 ou un polypeptide selon la revendication 3 ou un RCT tel que défini dans la revendication 12.

17. Composition pharmaceutique comprenant un peptide tel que défini dans la revendication 1 ou un polypeptide selon la revendication 3 ou un polynucléotide selon la revendication 16 ou des cellules T spécifiques d'*Aspergillus* telles que définies dans la revendication 11 et un vecteur pharmaceutiquement acceptable.

18. Peptide selon la revendication 1 ou polypeptide selon la revendication 3 ou polynucléotide selon la revendication 16, pour utilisation en médecine, de préférence dans lequel l'individu à traiter est un individu humain qui porte la HLA-DRB1*04 de classe II, par exemple pour utilisation pour combattre une infection par *Aspergillus,* ou pour combattre une allergie à *Aspergillus.*

19. Vaccin contre une infection par *Aspergillus* comprenant un peptide tel que défini dans la revendication 1 ou un polypeptide selon la revendication 3 ou un polynucléotide selon la revendication 16.

20. Cellule présentant l'antigène, telle qu'une cellule dendritique, qui présente un peptide tel que défini dans la revendication 1 ou un polypeptide selon la revendication 3, de préférence dans lequel la cellule présentant l'antigène porte la HLA-DRB1*04 de classe II.

21. Cellule présentant l'antigène selon la revendication 20, pour utilisation en médecine, éventuellement comme un vaccin contre une infection par *Aspergillus,* ou éventuellement pour utilisation pour combattre une infection par *Aspergillus* chez un patient, de préférence dans laquelle le patient porte la HLA-DRB1*04 de classe II.

22. Anticorps qui reconnaît sélectivement le peptide FHTYTIDWTKDAVTW lorsqu'il est présenté par la HLA-DRB1*04 de classe II.

23. Anticorps selon la revendication 22, pour utilisation en médecine, tel que pour utilisation pour combattre une infection par *Aspergillus,* de préférence dans lequel le receveur porte la HLA-DRB1*04 de classe II.

24. Procédé *in vitro* pour déterminer si un individu est infecté par *Aspergillus,* le procédé comprenant le fait de déterminer si un individu contient des cellules T spécifiques *d'Aspergillus* telles que définies dans la revendication 11, dans lequel l'individu porte la HLA-DRB1*04 de classe II.

25. Procédé *in vitro* pour déterminer si un individu est infecté par *Aspergillus,* le procédé comprenant l'utilisation d'un RCT selon la revendication 12, de préférence dans lequel l'individu porte la HLA-DRB1*04 de classe II.
